# EUROPEAN PATENT SPECIFICATION

(11) **EP 0 826 773 B1**
(45) Date of publication and mention of the grant of the patent: **26.01.2005**
(21) Application number: 97115289.7
(22) Date of filing: 03.09.1997
(51) Int. Cl.: C12N 15/53, C12N 1/21, C12N 15/52, C12P 13/24

(54) **Process for producing trans-4-hydroxy-l-proline**
Verfahren zur Herstellung von trans-4-Hydroxy-L-Prolin
Procédé de préparation de trans-4-hydroxy-L-proline

(30) Priority: 03.09.1996 JP 23272496
(43) Date of publication of application: 04.03.1998
(73) Proprietor: KYOWA HAKKO KOGYO CO., LTD., Chiyoda-ku, Tokyo 100 (JP)
(72) Inventor: Ozaki, Akio, Machida-shi, Tokyo 194 (JP); Shibasaki, Takeshi, Hofu-shi, Yamaguchi 747 (JP); Mori, Hideo, Machida-shi, Tokyo 194 (JP); Maruyama, Akihiko, Tokyo 168 (JP); Motoyama, Hiroaki, Yokohama-shi, Kanagawa 225 (JP)
(74) Representative: Vossius, Volker, Dr.

(56) References cited:
- EP-A- 0 146 929
- EP-A- 0 555 475
- EP-A- 0 641 862

## Description

### Field of the Invention

The present invention relates to a process for producing trans-4-hydroxy-L-proline using a non human transformant which contains a gene involved in L-proline-4-hydroxylase and has a reinforced proline biosynthesis activity. Trans-4-hydroxy-L-proline is useful as a starting compound for medicines such as carbapenem antibiotic and N-acetyl hydroxy proline using for antiphlogistic, and an additive to foods.

### Background of the Invention

The following processes are known as a method for producing trans-4-hydroxy-L-proline using microorganisms.
1) A process in which trans-4-hydroxy-L-proline is produced from 4-hydroxy-2-oxoglutaric acid using microorganisms of the genus Escherichia (Japanese Published Unexamined Patent Application No. 266,995/91)
2) A process in which trans-4-hydroxy-L-proline is produced directly through fermentation using bacteria or fungi (EP 0 547 898 A2, and Japanese Published Unexamined Patent Application Nos. 236,980/93 and 245,782/94)
3) A process in which trans-4-hydroxy-L-proline is produced from L-proline using microorganisms of the genus Streptomyces [J. Biol. Chem., 254, 6684 (1979), Biochem. Biophys. Res. Comm., 120, 45, (1984), Tetrahedron Letters , 34, 7489 (1993), and Tetrahedron Letters, 35, 4649 (1994)].
4) A process for producing trans-4-hydroxy-L-proline by conversion of L-proline in the presence of an enzyme source which is derived from a microorganism belonging to the genus *Dactylosporangium* or *Amycolatopsis* and which catalyses the hydroxylation of L-proline into trans-4-hydroxy-L-proline, a divalent ion and 2-ketoglutaric-acid (European patent publication No. EP 0 641 862).
5) A process for producing 4-hydroxy-L-proline by the conversion of 4-hydroxy-2-oxoglutaric acid in an aqueous medium in the presence of an amino group donor and an enzyme source belonging to the genus *Escherichia* and capable of converting 4-hydroxy-2-oxoglutaric acid into 4-hydroxy-L-proline (European patent publication No. EP 0 555 475).

Finally, a method is described, wherein a novel microorganism of the genus *Serratia* is used for the production of L-proline (European patent publication No. EP 0 146 929).

The conventional processes can hardly be performed on an industrial scale for the following reasons:
1) A substrate for producing trans-4-hydroxy-L-proline, such as 4-hydroxy-2-oxoglutaric acid is too expensive and is difficult to obtain.
2) The productivity of trans-4-hydroxy-L-proline is low.
3) The activity of the enzymes that relate to the production of trans-4-hydroxy-L-proline is quite weak.

With respect to the enzyme that catalyzes the production of trans-4-hydroxy-L-proline, it was reported that L-proline-4-hydroxylases were purified from Dactylosporangium sp. RH1 [Japanese Published Unexamined Patent Application No. 322,885/95] and a microorganism of the genus Streptomyces as mentioned-above [Biochem. J., 313, 185 (1966)].

It was also reported that the gene coding for L-proline-4-hydroxylase, which had the activity of converting free L-proline into trans-4-hydroxy-L-proline in the presence of 2-ketoglutaric acid, was cloned from the mentioned-above Dactylosporangium sp. RH1, that plasmid pRH71 containing said L-proline-4-hydroxylase gene was obtained, that plasmid pTr2-4OH which was capable of expressing L-proline-4-hydroxylase under the control of tandem tryptophan promoter was constructed, and that said gene was expressed in E. coli [The Japan Society for Bioscience, Biotechnology and Agrochem., meeting in 1996, collected abstracts of lecture, p. 257].

A process in which trans-4-hydroxy-L-proline is produced industrially advantageously using L-proline-4-hydroxylase having a high level of activity has been in demand.

On the other hand, the enzyme which participates in proline biosynthetic pathway and the gene coding for said enzyme were elucidated in E. coli, etc. [J. Gen. Microbiol., 118, 287 (1980); Biochim. Biophys. Acta., 104, 397 (1965)].

It was also known that proline biosynthesis was regulated by γ-glutamyl kinase, which is the first enzyme participates in proline biosynthetic pathway, and being subjected to feedback inhibition with proline in E. coli, etc. [Biochim. Biophys. Acta., 192, 462 (1969)].

Moreover, it was known that GK, which was desensitized to the feedback inhibition with proline, was constructed by mutating the gene, proB, coding for GK, and that a microorganism producing L-proline effectively was obtained using said mutated gene [Mol. Gen. Genet., 182, 82 (1981); J. Bacteriol., 156, 1249 (1983); J. Bacteriol., 164, 1088 (1985)]. As the mutated proB gene, proB74 was known. It was known that mutation of proB74 was carried out by substitution of G, which was 319th nucleotide from 5' terminal of proB coding region, with A, that is, substitution of aspartic acid, which was 107th amino acid from N-terminal of proB protein, to asparagine [Gene, 64, 199 (1988)].

The enzymes participating in proline decomposition, the genes coding for these enzymes and the regulation of these genes are also well known by advanced research in E. coli, the genus Salmonella, etc. [J. Bacteriol., 146, 895 (1981); J. Mol. Biol., 148, 895 (1981)].

Heretofore, a process for producing trans-4-hydroxy-L-proline using a microorganism having a reinforced proline biosynthesis activity has not been known.

The object of the present invention is to provide an efficient process for the production of trans-4-hydroxy-L-proline on the industrially applicable basis, and the additional object of the present invention is to provide a novel non human transformant which catalyzes the hydroxylation of L-proline at the 4-position of L-proline and which is useful in the above process.

The object of the present invention is to provide a non human transformant carrying a recombinant DNA which contains a gene coding for L-prolin-4-hydroxylase and is capable of expressing said gene at high efficiency, and having a reinforced proline biosynthesis activity for producing trans-4-hydroxy-L-proline industrially advantageously in a process for producing trans-4-hydroxy-L-proline efficiently using L-prolin-4-hydroxylase, and a process for producing trans-4-hydroxy-L-proline industrially at low cost using the transformant.

### Summary of the Invention

The present invention provides a non-human transformant having a vector comprising a DNA fragment that contains a gene coding for a protein which has the enzymatic activity of hydroxylating the 4-position of L-proline and which acts on free L-proline in the presence of 2-ketoglutaric acid and divalent iron ions to produce trans-4-hydroxy-L-proline, and wherein the transformant comprises an increased mumber of copies of a gene, coding for an enzyme participating in the biosynthesis of L-proline, wherein a proline biosynthesis-gene which codes for an enzyme that is subjected to feedback inhibition with proline has been mutated to reduce the feedback inhibition with proline, or wherein the proline decomposition activity has been removed from the host to reinforce the proline biosynthesis activity and a process for the production of trans-4-hydroxy-L-proline which comprises cultivating said transformant in a medium, allowing L-proline to coexist with 2-ketoglutaric acid, a divalent iron ion, in the presence of cells of the cultivated transformant as enzyme source, to convert L-proline into trans-4-hydroxy-L-proline, and recovering the trans-4-hydroxy-L-proline from the aqueous medium.

### Brief Description of Drawings

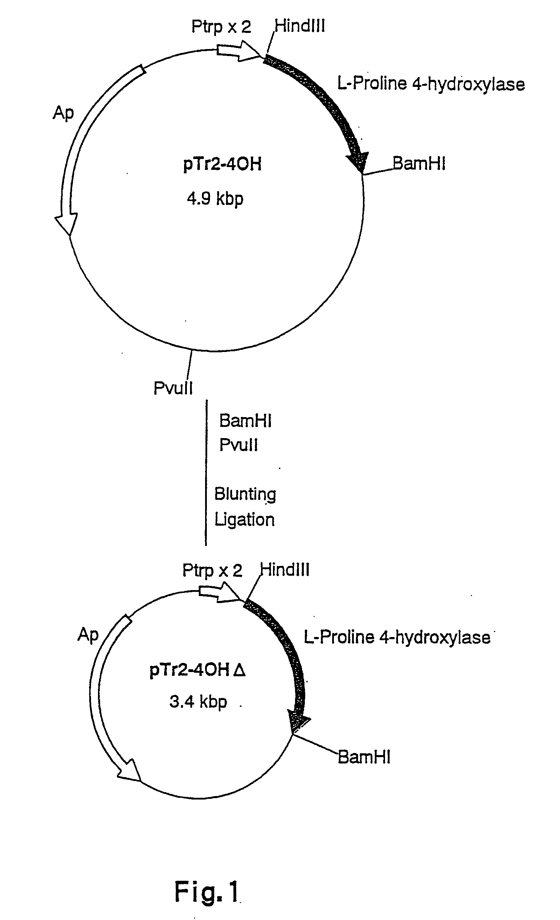

Fig. 1 shows the steps of constructing plasmid pTr2-4OHΔ.
   In the figure, the thick, solid black lines each indicate a part that contains an L-proline 4-hydroxylase gene. Ap indicates a pBR322-derived ampicillin-resistant gene; and Ptrpx2 indicates a promoter composed of two promoters of Escherichia coli-derived tryptophan operon as connected in series (tandem tryptophan promoter). The arrows each indicate the direction in which the gene is transcribed and translated. In the figure, only the restriction enzyme sites having relation to the construction of the plasmid are shown.

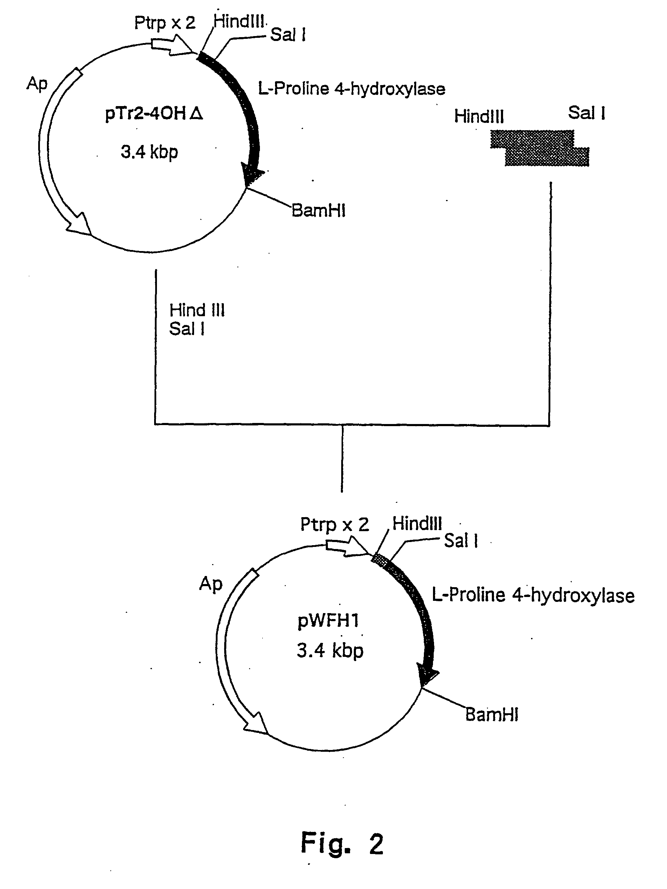

Fig. 2 shows the steps of constructing plasmid pWFH1.
   In the figure, the thick, shadowed lines each indicate a site into which a PCR-amplified fragment as treated with Hind III and SalI is inserted. The thick, solid black lines each indicate a part that contains a Dactylosporangium sp. RH1-derived L-proline 4-hydroxylase gene. Ap indicates a pBR322-derived ampicillin-resistant gene; and Ptrpx2 indicates a promoter composed of two promoters of Escherichia coli-derived tryptophan operon as connected in series (tandem tryptophan promoter). The arrows each indicate the direction in which the gene is transcribed and translated. In the figure, only the restriction enzyme sites having relation to the construction of the plasmid are shown.

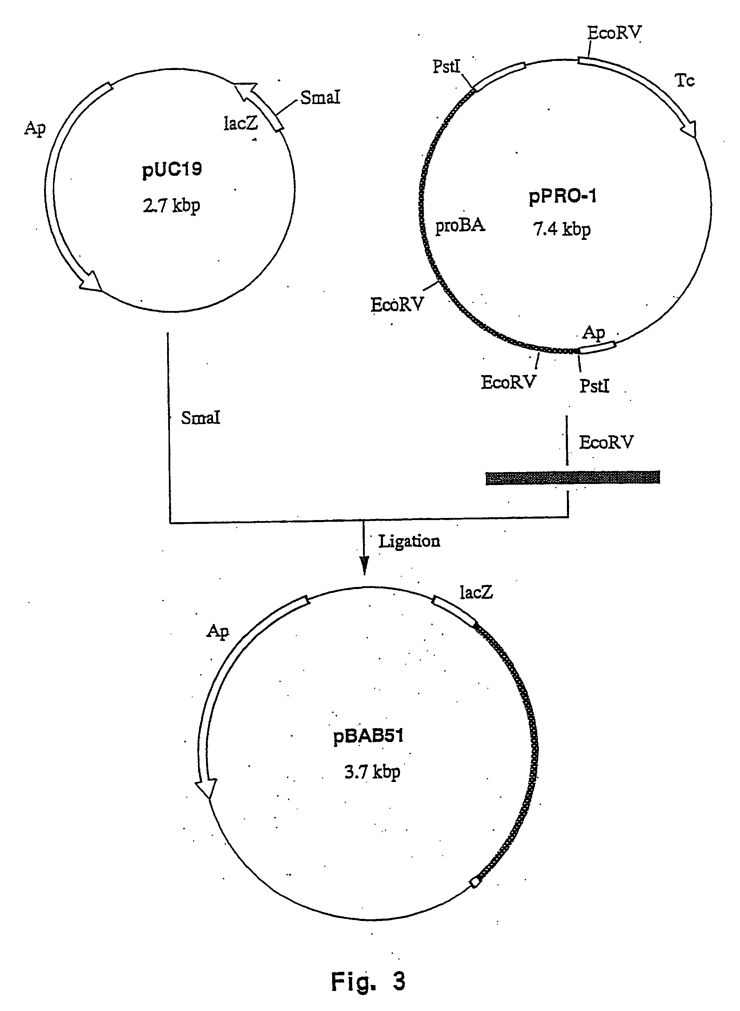

Fig. 3 shows the steps of constructing plasmid pBAB51.
   In the figure, the thick, shadowed lines each indicate proline biosynthesis-genes proB74 and proA. Ap indicates a pBR322-derived ampicillin-resistant gene; lacZ indicates β-galactosidase α fragment construction gene; and Tc indicates a pBR322-derived tetracycline-resistant gene. The arrows each indicate the direction in which the gene is transcribed and translated. In the figure, only the restriction enzyme sites having relation to the construction of the plasmid are shown.

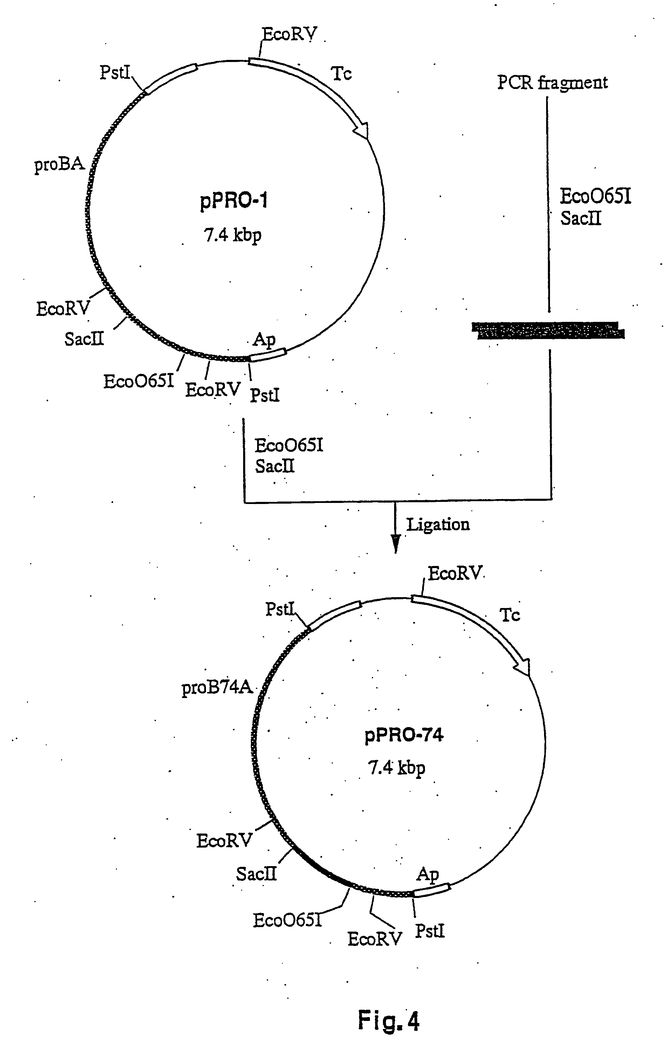

Fig. 4 shows the steps of constructing plasmid pPRO74.
   In the figure, the thick, shadowed lines each indicate proline biosynthesis-genes proB74 and proA. The thick, solid black lines in the thick, shadowed lines indicate proB74 gene containing one base pair which is different from proB gene. Tc indicates a pBR322-derived tetracycline-resistant gene. The arrows each indicate the direction in which the gene is transcribed and translated. In the figure, only the restriction enzyme sites having relation to the construction of the plasmid are shown.

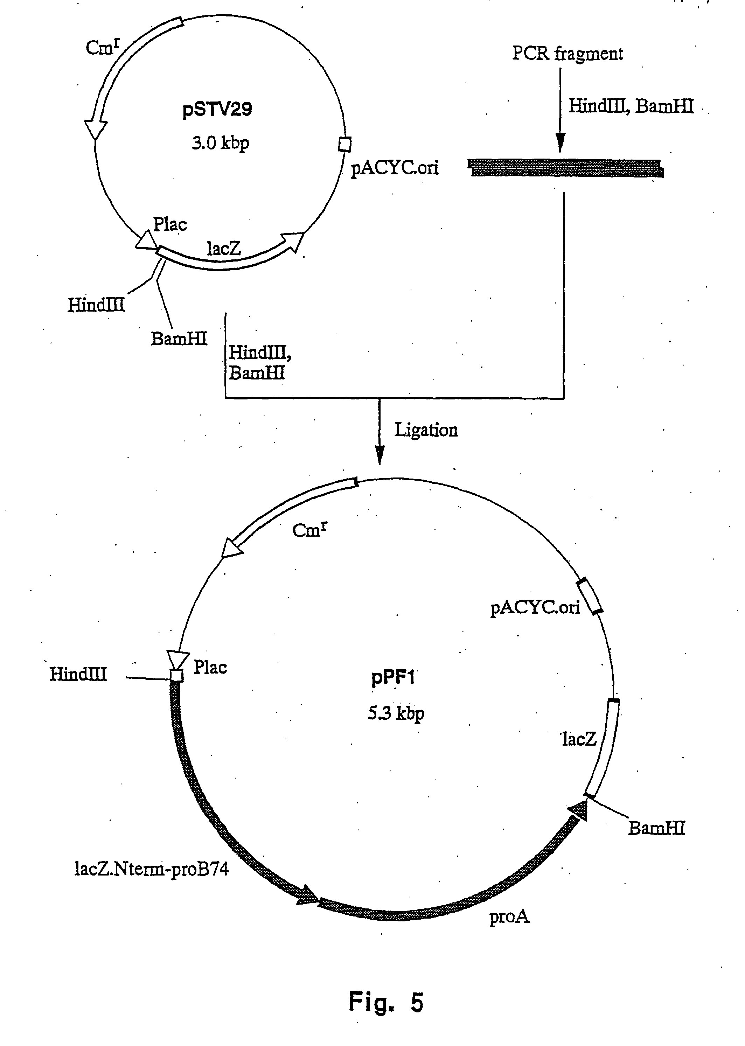

Fig. 5 shows the steps of constructing plasmid pPF1.
   In the figure, the thick, shadowed lines each indicate proline biosynthesis-genes proB74 and proA. Cm^{r} indicates Tn9-derived chloramphenicol resistant gene; pACYC.ori indicates pACYC184-derived replication origin; Plac indicates lac promoter; lacZ indicates β-galactosidase α fragment construction gene; and lacZ.Nterm-proB74 indicates the gene coding for a protein which unites N-terminal amino acids of β-galactosidase α fragment with a protein encoded by proB74. The arrows each indicate the direction in which the gene is transcribed and translated. In the figure, only the restriction enzyme sites having relation to the construction of the plasmid are shown.

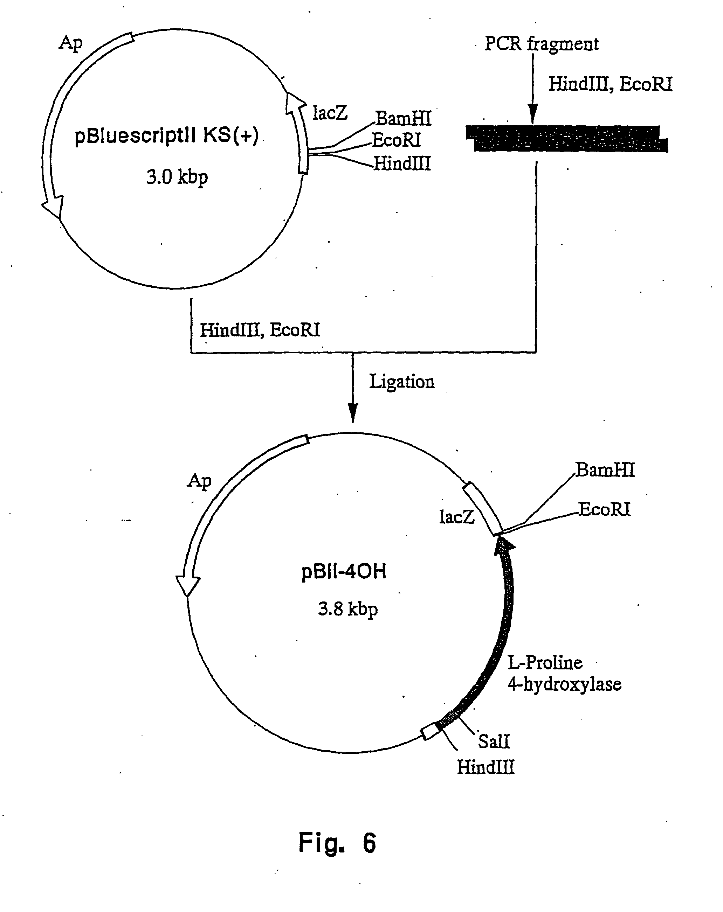

Fig. 6 shows the steps of constructing plasmid pBII-4OH.
   . In the figure, the thick, solid black lines each indicate a part that contains an L-proline 4-hydroxylase gene. Ap indicates a pBR322-derived ampicillin-resistant gene; and lacZ indicates β-galactosidase α fragment construction gene. The arrows each indicate the direction in which the gene is transcribed and translated. In the figure, only the restriction enzyme sites having relation to the construction of the plasmid are shown.

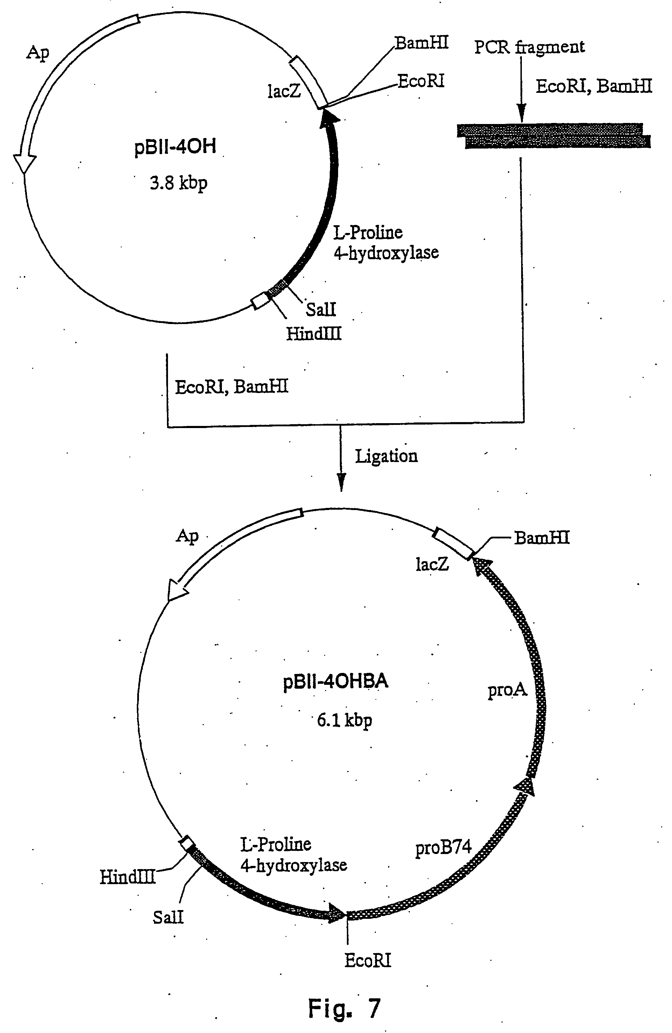

Fig. 7 shows the steps of constructing plasmid pBII-4OHBA.
   In the figure, the thick, solid black lines each indicate a part that contains an L-proline 4-hydroxylase gene. Ap indicates a pBR322-derived ampicillin-resistant gene; and lacZ indicates β-galactosidase α fragment construction gene. The arrows each indicate the direction in which the gene is transcribed and translated. In the figure, only the restriction enzyme sites having relation to the construction of the plasmid are shown.

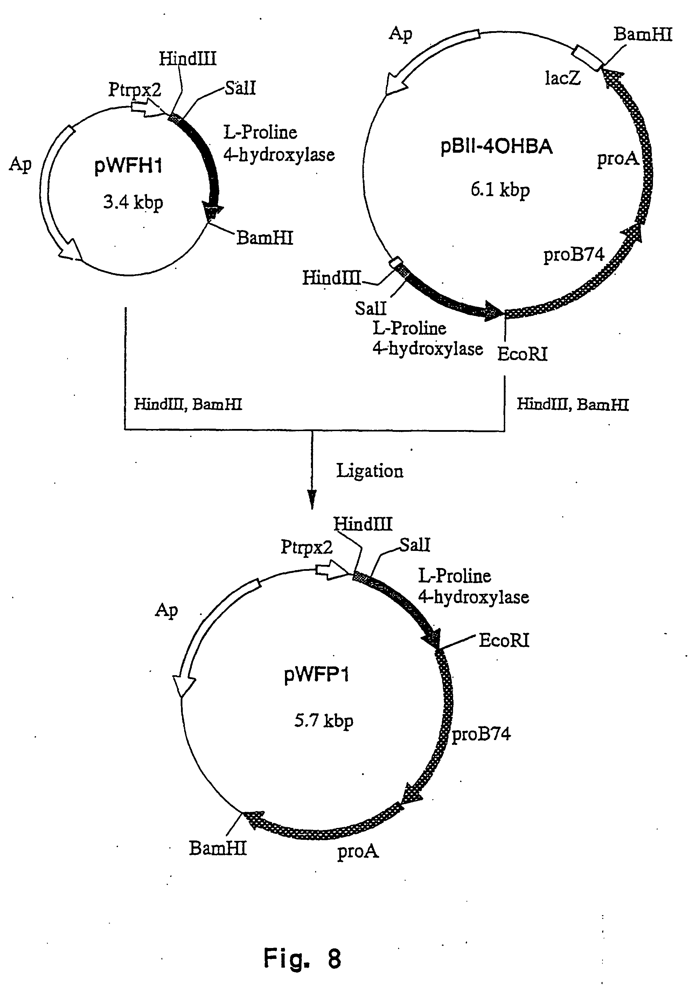

Fig. 8 shows the steps of constructing plasmid pWFP1.
   In the figure, the thick, solid black lines each indicate a part that contains an L-proline 4-hydroxylase gene. The thick, shadowed lines each indicate proline biosynthesis-genes proB74 and proA. Ap indicates a pBR322-derived ampicillin-resistant gene; and lacZ indicates β-galactosidase a fragment construction gene. Ptrpx2 indicates a promoter composed of two promoters of Escherichia coli-derived tryptophan operon as connected in series (tandem tryptophan promoter). The arrows each indicate the direction in which the gene is transcribed and translated. In the figure, only the restriction enzyme sites having relation to the construction of the plasmid are shown.

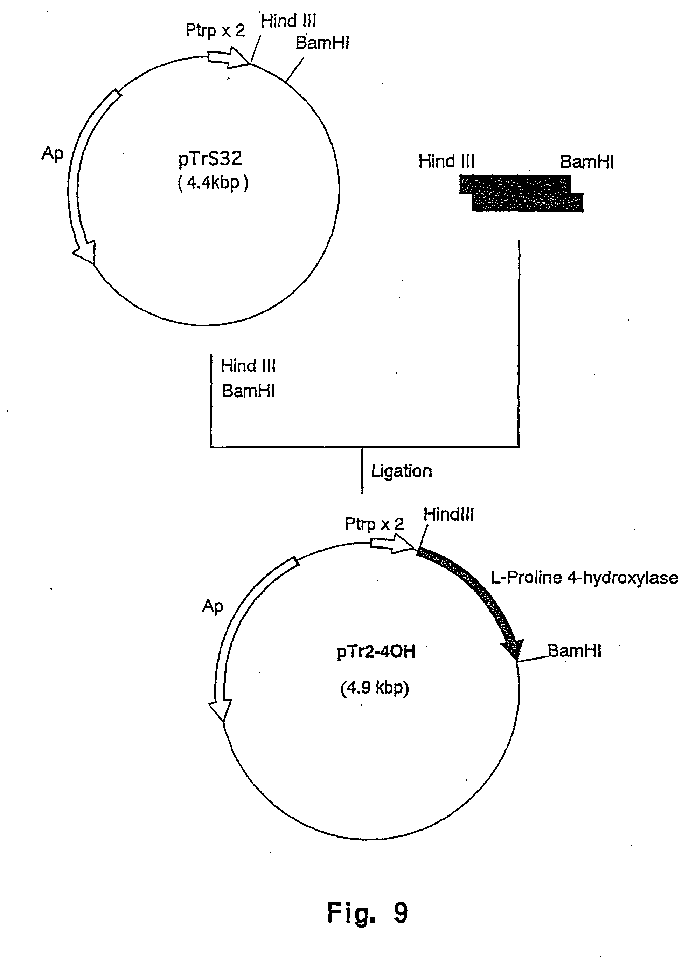

Fig. 9 shows the steps of constructing plasmid pTr2-4OH.
   In the figure, the thick, solid black line each indicate a part that contains an L-proline 4-hydroxylase gene. Ap indicates a pBR322-derived ampicillin-resistant gene; and Ptrpx2 indicates a promoter composed of two promoters of Escherichia coli-derived tryptophan operon as connected in series (tandem tryptophan promoter). The arrows each indicate the direction in which the gene is transcribed and translated. In the figure, only the restriction enzyme sites having relation to the construction of the plasmid are shown.

### Detailed Description of the Invention

The L-proline-4-hydroxylases are enzymes which catalyzes the reaction in which free L-proline is hydroxylated in the presence of 2-ketoglutaric acid and a divalent ion to form trans-4-hydroxy-L-proline.

As the plasmids containing the DNA encoding the L-proline-4-hydroxylase, for example, pRH71, etc. can be mentioned. Escherichia coli SOLR/pRH71 which is Escherichia coli SOLR containing pRH71 has been deposited with the National Institute of Bioscience and Human-Technology of the Agency of Industrial Science and Technology at 1-3, Higashi 1-chome, Tsukuba-shi, Ibaraki-ken 305, Japan as of March 2, 1995 under FERM BP-5025 in terms of the Budapest Treaty.

To express the thus-obtained L-proline-4-hydroxylase gene in the host, the DNA fragment containing the L-proline-4-hydroxylase gene is first cleaved by a restriction endonuclease or other deoxyribonuclease to form a DNA fragment of a suitable length containing the L-proline-4-hydroxylase gene. The thus-formed DNA fragment is inserted into an expression vector at the downstream position of the promoter therein, and thereafter the expression vector having the thus-inserted DNA therein is introduced into a host cell suitable for the expression vector.

Any host cell can be used, so long as the intended gene can be expressed in the host cell. As examples of the host cell, microbial cells of a microorganism belonging to the genus Escherichia, Serratia, Corynebacterium, Brevibacterium, Pseudomonas, and Bacillus, etc., as well as yeast strains, animal cell hosts, etc. can be mentioned.

An expression vector, which is autonomously replicable in the above-mentioned host cell or capable of being inserted into a chromosome and which contains a promoter at the position where the L-proline-4-hydroxylase gene can be transcribed, can be used.

When the microorganisms such as Escherichia coli or the like are used as the host cell, it is advisable that the expression vector is replicated autonomously in the microorganisms and is composed of a promoter, a ribosome binding sequence such as a Shine-Dargarno sequence, an L-proline-4-hydroxylase gene and a transcription termination sequence. A regulatory gene may be contained therein.

As examples of the expression vector, mentioned are pBTrp2, pBTac1, pBTac2 (all commercially available from Boehringer Manheim Co.); pKYP10 (see Japanese Published Unexamined Patent Application No. 110600/83); pKYP200 [see Agric. Biol. Chem., 48, 669 (1984)]; pLSA1 [see Agric. Biol. Chem., 53, 277 (1989)]; pGEL1 [see Proc. Natl. Acad. Sci. USA., 82, 4306 (1985)]; pBluescript (produced by STRATAGENE Co.); pTrs30 [prepared from Escherichia coli JM109/pTrS30 (FERM BP-5407); pTrs32 [prepared from Escherichia coli JM109/pTrs32 (FERM BP-5408)], etc.

As the promoter, usable is any one capable of being expressed in hosts such as Escherichia coli. For example, mentioned are promoters derived from Escherichia coli, phage, etc., such as trp promoter (Ptrp), lac promoter (Plac), P_{L} promoter and P_{R} promoter. Also usable are artificially designed and modified promoters, such as Ptrpx2 to be prepared by connecting two Ptrps in series, as well as tac promoter (ptac).

As the ribosome-binding sequence, any one capable of being expressed in hosts such as Escherichia coli can be used. However, it is desirable to use plasmids having a ribosome-binding sequence and an initiation codon as spaced at suitable intervals therebetween (for example, by 6 to 18 bases).

The L-proline-4-hydroxylase gene includes any and every gene that codes for an L-proline-4-hydroxylase. However, it is desirable that the bases constituting the DNA sequence of the gene are suitably substituted in order that the substituted DNA sequence can be composed of codons most suitable for expression in the host microorganisms to be used. As examples of L-proline-4-hydroxylase genes where the constitutive bases have been substituted to modify them into codons most suitable for their expression, mentioned are the nucleotide sequence of Sequence No. 1, etc.

Transcription terminator sequences are not always necessary for the expression of the genes of the present invention. However, it is desirable that a transcription terminator sequence is arranged just after the structural gene.

Examples of the host cells usable in the present invention include Escherichia coli XL1-Blue, Escherichia coli XL2-Blue, Escherichia coli DH1, Escherichia coli MC1000, Escherichia coli KY3276, Escherichia coli W1485, Escherichia coli JM109, Escherichia coli HB101, Escherichia coli No. 49, Escherichia coli W3110, Escherichia coli NY49, Bacillus subtilis, Bacillus amyloliquefacines, Brevibacterium immariophilum ATCC14068, Brevibacterium saccharolyticum ATCC14066, Brevibacterium flavum ATCC14067, Brevibacterium lactofermentum ATCC13869, Corynebacterium glutamicum ATCC13032, Corynebacterium acetoacidophilum ATCC13870, Microbacterium ammoniaphilum ATCC15354, etc.

When the yeast strain is used as the host cell, for example, YEp13 (ATCC37115), YEp24 (ATCC37051), YCp5O (ATCC37419), etc. can be used as the expression vector.

As the promoter, any one that can be expressed in the host cell of the yeast strain can be used. As examples of the promoters, promoters of glycolytic genes such as hexose kinase, gal 1 promoter, gal 10 promoter, heat shock protein promoter, MFαl promoter, and CUP 1 promoter can be used.

As examples of the host cells, Saccharomyces cerevisae, Schizosaccharomyces pombe, Kluyveromyces lactis, Trichosporon pullulans, and Schwanniomyces alluvius, etc. can be mentioned.

When the animal cells are used as the host cell, for example, pcDNA I/Amp, pcDNA I and pcDM8 (all commercially available from Funakosi Co.), etc. can be used as the expression vector.

As the promoter, any one that can be expressed in the host cell of animal cells can be used. For example, a promoter of an IE (immediate early) gene of human CMV, etc. can be used. An enhancer of the IE gene of human CMV may be used together along with the promoter.

As examples of the host cells, Namalwa, HBT5637 (Japanese Published Unexamined Patent Application No. 299/88), COS-cell, CHO-cell, etc. can be fused.

To introduce DNA into animal cells in vitro, any and every method capable of introducing DNA into animal cells can be employed herein. For example, employable are electroporation methods [see Miyaji et al., Cytotechnology, 3, 133 (1990)], calcium phosphate methods (see Japanese Published Unexamined Patent Application No. 227075/90), lipofection methods [see Philip L. Felgner, et al., Proc. Natl. Acad. Sci., USA, 84, 7413 (1987)], etc. The resulting transformants can be collected and cultivated in accordance with the methods described in Japanese Published Unexamined Patent Application Nos. 227075/90 and 257891/90.

To reinforce the proline biosynthesis activity of the hosts, employable are a means of increasing the number of copies of the gene that codes for an enzyme participating in the biosynthesis of L-proline (hereinafter referred to as "proline biosynthesis-gene") in the hosts, a means of mutating a proline biosynthesis-gene that shall be subjected to feedback inhibition with proline to produce a mutant gene that codes for an enzyme which participates in proline biosynthesis, to which the feedback inhibition with proline is greatly reduced (hereinafter referred to as "proline biosynthetase"), followed by introducing the resulting mutant gene into the hosts, a means of removing the proline decomposition activity from the hosts, and also a combination of any of these means.

The proline biosynthesis-gene, or the mutant gene that codes for a proline biosynthetase can exist on the chromosomes of the hosts or can also exist on the vectors, such as plasmids, in the hosts.

Any gene can be used so long as the gene codes for a proline biosynthetase. As the gene coding for proline biosynthetic enzyme which is desensitized remarkably to the feedback inhibition with proline, for example, it includes a gene proB74 such as that mentioned hereinabove, a gene DHP^{r}proB [Gene, 39, 109 (1984)], etc.

In the case of coexistence of any of the proline biosynthesis-gene and the mutant gene which codes for a proline biosynthetase with an L-proline-4-hydroxylase gene on vectors such as plasmids in the same host, both the genes can exist either on a single plasmid or on plural, co-existable plasmids.

For the plasmids of E. coli, for example, the combination of such co-existable plasmids include a colicin E1 family plasmid (e.g., pBR322) and a pACYC family plasmid; a colicin E1 family plasmid and an F-factor family plasmid; and a colicin family E1 plasmid and an R-factor family plasmid.

To express the proline biosynthesis-gene in the host, employable is the same process as that mentioned hereinabove for the expression of L-proline-4-hydroxylase gene.

The host which loses proline decomposition activity can be obtained by selecting the strain which forms white colony on the Pro-TTC plate [Appl. Environ. Microbiol., 33, 434 (1977)] after processing the host with the mutagen.

It is known that E. coli shall lose its ability of assimilating proline if a transposon is inserted into a suitable site of its put gene [Genetics, 92, 741 (1979)]. For E. coli, therefore, a transposon is introduced thereinto, and a mutant E. coli that has lost its proline decomposition activity also can be selected through a chemical resistance test and a cell-growing test on a Pro-TTC plate.

The mutant that has lost its proline decomposition activity due to the introduction of a transposon thereinto can be subjected to P1 transduction [A Short Course In Bacterial Genetics, A Laboratory Manual and Handbook for Esherichia coli and Related Bacteria, J. H. Miller, Cold Spring Harbor Laboratory Press, 1922, Laboratory Manual, pp. 263 ] to thereby transfer its characteristic not having a proline decomposition activity into a different strain.

The non human transformant, which is obtained from a host cells having reinforced proline biosynthesis activity, is cultivated by an ordinary cultivation method.

The medium for cultivating these microbial transformants such as Escherichia coli, yeast strains or the like may be any of natural media and synthetic media that contain carbon sources, nitrogen sources, inorganic salts, etc. that may be assimilated by the microorganisms.

Any carbon sources that can be assimilated by the microorganisms may be used. Examples of the carbon source include carbohydrates such as glucose, fructose, sucrose, molasses containing these components, starch and starch hydrolyzates; organic acids such as acetic acid and propionic acid; and alcohols such as ethanol and propanol.

As the nitrogen sources, ammonia, ammonium salts of inorganic and organic acids such as ammonium chloride, ammonium sulfate, ammonium acetate and ammonium phosphate, other nitrogen-containing compounds, peptone, meat extracts, yeast extracts, corn steep liquor, casein hydrolyzates, soybean cakes, soybean cake hydrolyzates, cultured fermented cells, their digested products, etc. may be used.

As inorganic salts, potassium dihydrogen phosphate, dipotassium hydrogen phosphate, magnesium phosphate, magnesium sulfate, sodium chloride, ferrous sulfate, manganese sulfate, copper sulfate, calcium carbonate, etc. may be used.

The cultivation is conducted under aerobic conditions, for example, with shaking culture or submerged-aerial stirring culture. The temperature for the cultivation is 15 to 40°C. The period for the cultivation is usually 16 to 100 hours. During the cultivation, the pH of the medium is kept at 3.0 to 9.0. The pH is adjusted using inorganic or organic acids, alkaline solutions, urea, calcium carbonate, ammonia or the like.

Antibiotics such as ampicillin, tetracycline or the like may be added to the medium during the cultivation, if required.

For the cultivation of the microorganisms which are transformed with the expression vector using the inducible promoter, inducers may be added to the medium, if required. For example, in cultivation of microorganisms transformed with the expression vector using lac promoter, isopropyl-β-D-thiogalactopyranoside (IPTG) may be added to the medium. In cultivation of microorganisms transformed with the expression vector using trp promoter, indoleacrylic acid (IAA) may be added to the medium.

As the medium for cultivating the non human transformants which are obtained by using the animal cells as a host cell, RPMI1640 medium and Eagle's MEM medium which are generally used or these culture media containing a fetal bovine serum can be used.

The cultivation of the cells is conducted in the presence of 5% CO₂. The temperature for the cultivation is preferably 35 to 37°C, and the period for the cultivation is usually 3 to 7 days.

Antibiotics such as kanamycin, penicillin or the like may be added to the medium during the cultivation, if required.

A considerable amount of L-proline-4-hydroxylase is produced and accumulated in the thus-cultivated transformants in comparison with recombinant E. coli containing pTr2-4OH and the microorganism strain used as the gene source, such as Dactylosporangium sp. RH1 or the like. Thus, the isolation and purification of the enzyme or the production of trans-4-hydroxy-L-proline from L-proline using the enzyme can be performed far more efficiently in comparison with the production of trans-4-hydroxy-L-proline from L-proline using the non genetically-engineered microorganism as the gene source, such as Dactylosporangium sp. RH1 or the like.

The production of L-proline-4-hydroxylase in the transformants can be carried out by adding the culture, or the cells to an aqueous medium suitable for the enzymatic reaction together with L-proline, a divalent iron ion and 2-ketoglutaric acid, and adding a surfactant or an organic solvent, if required, to determine trans-4-hydroxy-L-proline produced. With respect to the activity of the L-proline-4-hydroxylase of which is formed in the cell, the activity of the enzyme for producing 1 nmol of trans-4-hydroxy-L-proline for 1 minute under the following conditions is defined as 1 unit (U). The microorganism cells and the animal cells are here referred to the cells.

### Measurement of L-proline-4-hydroxylase activity:

The cells, the treated cells or the enzyme preparation are added to 240 mM MES [2-(N-morphorino)ethanesulfonic acid] buffer containing 12 mM L-proline, 24 mM 2-ketoglutaric acid, 4 mM ferrous sulfate and 8 mM L-ascorbic acid to make 250 µl in total. The mixture is kept at 35°C for 10 minutes. The reaction mixture is heated at 100°C for 2 minutes to stop the reaction, and the amount of trans-4-hydroxy-L-proline produced in the reaction mixture is determined by high performance liquid chromatography (hereinafter referred to as HPLC).

For the determination, any method capable of determining the amount of trans-4-hydroxy-L-proline may be employed. For example, generally usable are a post-column derivatization method where trans-4-hydroxy-L-proline in the reaction mixture is separated and eluted using ligand exchange chromatography column such as SUMCHIRAL OA5000 produced by Sumika Chemical Analysis Service Limited, etc., subsequently, trans-4-hydroxy-L-proline is derivatized with 7-chloro-4-nitrobenz-2-oxa-1,3-diazole (hereinafter referred to as NBD), and then derivatized trans-4-hydroxy-L-proline is detected, and a pre-column derivatization method where the compound to be determined in the reaction mixture is previously reacted with NBD to form its NBD-derivative, the derivative is separated by reversed-phase chromatography using HPLC and the thus-separated derivative is detected [Analytical Biochemistry, 138, 390 (1984)]. Detect of NBD-derivative is carried out by measuring fluorescence of its NBD-derivative (excitation wavelength: 503 nm, emission wavelength: 541 nm).

The cultivated transformant cells that have been identified to contain the L-proline-4-hydroxylase as formed therein can be cultivated under the same conditions as above, under which the transformant cells were cultivated, to thereby make the cells produce and accumulate trans-4-hydroxy-L-proline in the cells, and the thus-produced trans-4-hydroxy-L-proline can be collected from the culture.

If desired, 2-ketoglutaric acid and divalent iron ions may be added to the media during the cultivation of the transformant cells.

The trans-4-hydroxy-L-proline produced by the present invention can be determined quantitatively by the above-mentioned post-column derivatization method or pre-column derivatization method.

The present invention is illustrated more specifically by referring to the following Examples.

### Example 1 : Construction of L-proline-4-hydroxylase high expression plasmid

A DNA as indicated in Sequence No. 2 and a DNA as indicated in Sequence No. 3 were synthesized, using 380A·DNA Synthesizer (produced by Applied Biosystems Co.). These DNAs were so designed that their 3' terminals of 25 bp are complementary to each other. These DNAs each have a nucleotide sequence coding for the N-terminal site of Dactylosporangium sp. RH1-derived L-proline-4-hydroxylase protein, in which the nucleotide sequence has been site-specifically substituted to make it a codon that is the most suitable in its expression in Escherichia coli.

Using these synthetic DNA's as primers and templates, PCR was conducted. The reaction was conducted, using 20 µl of a reaction mixture comprising 0.5 U of Pfu DNA polymerase (produced by STRATAGENE Co.), 2 µl of x10 buffer for Pfu DNA polymerase, 2 µl of DMSO, 1 µl of 2.5 mM dNTP, 2 µM of the synthetic DNA of Sequence No. 2 and 2 µM of the synthetic DNA of Sequence No. 3. The reaction mixture was incubated at 96°C for 5 minutes. Subsequently, a three step incubation, namely at 96°C for 2 minutes, at 50°C for 1 minute and at 75°C for 1 minute was repeated for a total of 35 times.

After the resulting reaction mixture was subjected to 15% polyacrylamide gel electrophoresis, the formation of an amplified fragment of 107 bp was identified.

The amplified fragment was recovered from the gel using da Vinci Kun (Pen Touch Recovery NB-7000 Model) manufactured by Nippon Eido K.K. Then, both the terminals of the thus-recovered DNA fragment of 107 bp were cleaved with Hind III and Sal I, and the thus-processed fragment was recovered using MERmaid Kit (produced by Bio, Inc.).

The amount of the liquid thus recovered was 16 µl.

Plasmid pTr2-4OH DNA, which was constructed according to the method described in reference example 1, was cleaved with Bam HI and Pvu II. The reaction mixture was subjected to agarose gel electrophoresis, through which the formation of two fragments was identified. Of these, the longer fragment having the structural gene of L-proline-4-hydroxylase was isolated, using Prep-A-Gene (produced by Biorad Co.), and its terminals were blunted using a blunting kit (produced by Takara Shuzo Co.) and then cyclized using a ligation kit (produced by Takara Shuzo Co.).

With the thus-obtained plasmid, E. coli JM109 strain was transformed in a usual manner, and the resulting transformant cells were spread on LB-agar medium containing 50 µg/ml of ampicillin and then cultivated thereon overnight at 37°C.

A plasmid was extracted from the grown colonies of the transformant cells in a usual manner, and its structure was identified through digestion with restriction enzyme.

As a result of the above, obtained was plasmid pTr2-4OHΔ, which is lacking for a part of the sequence of pTr2-4OH (see Fig. 1).

Plasmid pTr2-4OHΔ DNA was cleaved with Hind III and Sal I. The PCR-amplified fragment that had been processed with Hind III and Sal I in the above was inserted into the Hind III-Sal I cleavage site of the plasmid, using a ligation kit (produced by Takara Shuzo Co.).

With the thus-obtained plasmid, E. coli XL1-Blue MRF' strain were transformed in a usual manner, and the resulting transformant cells were spread on LB-agar medium containing 50 µg/ml of ampicillin and then cultivated thereon overnight at 37°C.

A plasmid, pWFH1 was isolated from the grown colonies of the transformant cells in a usual manner.

Its structure was identified through digestion with various restriction enzymes. The part of the plasmid into which the PCR-amplified fragment had been inserted was sequenced, using a base sequencing kit (Taq DyeDeoxy™ Terminator Cycle Sequencing Kit, produced by Applied Biosystems Co.), to determine its nucleotide sequence. The determined nucleotide sequence is indicated by Sequence No. 1.

As a result of the above, it was revealed that plasmid pWFH1 contains the structural gene DNA fragment having for the amino acid sequence which is entirely the same as the Dactylosporangium sp. RH1-derived L-proline-4-hydroxylase except that from the 5'-terminal to the Sal I site of the structural gene is partly different from the Dactylosporangium sp. RH1-derived nucleotide sequence, in the same direction as the transcription direction of Ptrpx2 (see Fig. 2).

As is shown in Table 1 below, the transformants produced L-proline-4-hydroxylase by 1400 times/cell as much as the Dactylosporangium sp. RH1 strain which had been used as the gene source and by about 5.4 times/cell as much as the transformant containing pTr2-4OH.

**Table 1**

| Strain | Cell Activity¹⁾ | Relative Activity²⁾ |
|---|---|---|
| Dactylosporangium sp. RH1³⁾ | 0.028 | 1 |
| E. coli ATCC12435/pTr14⁴⁾ | 3.7 | 132 |
| E. coli ATCC12435/pWFH1⁴⁾ | 40 | 1400 |

| | | |
|---|---|---|
| 1) Cell activity indicates the enzymatic activity per mg of wet cells (U/mg wet cells). One U indicates the enzymatic activity of producing 1 nmol of trans-4-hydroxy-L-proline per minute (nmol/min). | | |
| 2) Relative activity is based on the enzymatic activity produced by Dactylosporangium sp. RH1 strain of being 1 (one). | | |
| 3) Cell activity was calculated from the amount of producing trans-4-hydroxy-L-proline described in Reference Example 2. | | |
| 4) Cell activity was calculated from the amount of producing trans-4-hydroxy-L-proline described in Example 8. | | |

### Example 2 : Construction of Strain Losing Proline Decomposition Activity:

A gene putA that participates in the proline decomposition in E. coli ATCC12435 was broken according to the method mentioned below to construct a strain losing proline decomposition activity.

Cells of a stock strain E. coli ME8395 [F^{―}: pyrD34, trp-45, his-68, thyA25, thi deoR33, galK35, xyl-7, mtl-2, malA1, rpsL118, λ^{R} (λ)^{―}, appA1, putA::Tn5 (Mu⁺)] available from the National Institute of Genetics were inoculated in an LB medium containing 35 µg/ml kanamycin, and cultivated overnight.

Then 100 µl of a solution of P1 phage was added to and mixed with 100 µl of the resulting culture, and left as it was for 5 minutes.

The resulting mixture was mixed with 3 ml of LB-soft agar medium containing 10 mM of CaCl₂, then layered over an LB-agar medium containing 10 mM of CaCl₂, and cultivated at 37°C for 7 hours.

After the cultivation, the phage lysate thus formed on the surface of the agar medium was collected in 2 ml of an LB medium containing 10 mM of CaCl₂.

To the liquid thus collected, 0.5 ml of chloroform was added, mixed therewith, using a Vortex mixer, and then centrifuged at 3000 rpm for 15 minutes. The resulting supernatant was used as a P1 phage lysate.

Then 50 µl of the P1 phage lysate liquid was mixed with 100 µl of a culture of E. coli ATCC12435 that had been cultivated in an LB medium containing 10 mM of CaCl₂, and then was kept standing at 37°C for 20 minutes.

The resulting liquid mixture was mixed with 3 ml of F-top-citrate (containing 8.5 g/l of NaCl, 100 mM of disodium citrate, and 0.7 % of agar), applied onto an LB-agar medium containing 35 pg/ml kanamycin, and cultivated at 37°C for one day.

The kanamycin-resistant cells thus grown through the cultivation were suspended in 0.85 % NaCl, then spread onto a Pro-TTC plate (comprising 7 g/l K₂HPO₄, 3 g/l KH₂PO₄, 0.1 g/l MgSO₄, 2 g/l proteose peptone, 0.025 g/l 2,3,5-triphenyltetrazolium chloride, 2 g/l L-proline and 15 g/l agar, pH 7.2), and cultivated at 37°C for 1 to 2 days.

The strain that had produced white colonies on the Pro-TTC plate through the cultivation was selected as a strain losing the activity of decomposing and assimilating proline. Thus was obtained a strain losing proline decomposition activity, E. coli WT1.

The strain WT1 has been deposited with National Institute of Bioscience and Human-Technology, Agency of Industrial Science and Technology at 1-3, Higashi 1-chome, Tsukuba-shi, Ibaraki-ken, 305 Japan as of August 7, 1996 under FERM BP-5618 in terms of the Budapest Treaty.

### Example 3 : Construction of Plasmid Expressing Proline Biosynthetic Genes proB74 and proA:

A plasmid pPF1 was constructed according to the method mentioned below, using the plasmid expressing a mutant gene proB74 (this was mutated from an E. coli-derived gene proB which codes for γ-glutamyl kinase, to be desensitized to the feedback inhibition with proline) and an E. coli-derived gene proA which codes for γ-glutamyl phosphate reductase.

A plasmid pPRO-1 containing E. coli-derived genes proA and proB [this was obtained from an E. coli K83 strain (FERM BP-2807)] was cleaved with Eco RV and then subjected to agarose gel electrophoresis, from which was obtained a DNA fragment of about 1 kb containing a part of the gene proB, using a Prep-A-gene DNA Purification System (produced by BIO-RAD Co.).

The DNA fragment was ligated with pUC19 (produced by Takara Shuzo Co.) that had been cleaved with SmaI, to obtain a plasmid pBAB51 (see Fig. 3).

The gene proB existing in the plasmid was mutated into a known, mutant gene proB74 as desensitized to the feedback inhibition with proline [A.M. Dandekar and S.L. Uratsu, J. Bacteriol. 170, 5943 (1988)], according to the method mentioned below.

An oligonucleotide A1 as indicated in Sequence No. 4 and an oligonucleotide A2 as indicated in Sequence No. 5 were synthesized, using a DNA synthesizer, 380A Model (produced by Applied Biosystems Co.).

A partial sequence of the mutant gene proB74 that had been mutated from proB was amplified through PCR, using a pair of primers, oligonucleotide A1 and M13 primer M3 (produced by Takara Shuzo Co. - Catalog No. 3831) and using pBAB51 as a template.

The PCR was conducted, using 20 µl of a reaction mixture comprising 0.1 µg of pBAB51, 2 µM of oligonucleotide A1, 2 µM of M13 primer M3, 1 U of Taq DNA polymerase (produced by Takara Shuzo Co.), 1.6 µl of dNTP mixture (produced by Takara Shuzo Co. - Catalog No. 4030) and 2 µl of an additive buffer. A three step incubation, namely at 94°C for 30 seconds, at 52°C for 30 seconds and at 72°C for 1 minute was repeated for a total of 30 times. Finally, the thus-incubated system was further incubated at 72°C for 5 minutes.

In the same manner as above, a partial sequence of the mutant gene proB was amplified through PCR, using a pair of primers, oligonucleotide A2 and M13 primer RV (produced by Takara Shuzo Co. - Catalog No. 3830A) and using pBAB51 as a template.

These two DNAs that had been amplified through such PCR were separately subjected to agarose gel electrophoresis and then purified using Prep-A-gene DNA Purification System (produced by BIO-RAD Co.).

In the same manner as above except using a mixture of the two pure DNA fragments as a template along with M13 primer M3 and M13 primer RV as primers, a DNA fragment of about 1 kb containing a nucleotide sequence of proB74 was obtained through the PCR and purification.

The DNA fragment was cleaved with Eco O65I and Sac II to obtain an Eco 065I-Sac II cleaved fragment.

This fragment was ligated with a DNA fragment (of about 6.8 kbp) as obtained from pPRO-1 through digestion with Eco O65I and Sac II to construct a plasmid pPRO74, which is different from pPRO-1 in that the proB gene of pPRO-1 has been substituted with the de-sensitized proB74 gene (see Fig. 4).

An oligonucleotide p1 of Sequence No. 6 and an oligonucleotide p2 of Sequence No. 7 were synthesized, using a DNA synthesizer, 380A^{.}Model (produced by Applied Biosystems Co.).

proB74 and proA were amplified through PCR, using these p1 and p2 as primers and using pPRO74 as a template.

The PCR was conducted, using 20 µl of a reaction mixture comprising 0.1 µg of pPRO74, 2 µM of p1, 2 µM of p2, 1 U Takara EX Taq (produced by Takara Shuzo Co. - Code RR001Q)) and 1.6 µl of dNTP mixture (produced by Takara Shuzo Co. - Catalog No. 4030). A three step incubation, namely at 94°C for 1 minute, at 42°C for 2 minute and at 73°C for 3 minute was repeated for a total of 30 times.

The resulting reaction mixture was subjected to agarose gel electrophoresis, through which was extracted an amplified fragment of 2370 bp containing proB74 and proA in a usual manner, and the DNA fragment was collected using GENECLEAN II KIT (produced by BIO 101, Inc.). The thus-collected DNA fragment of 2370 bp was cleaved with Hind III and Bam HI, and then an ethanol precipitate was obtained by ethanol precipitation. The ethanol precipitate was dissolved in 5 µl TE and used as a fragment having proB74 and proA.

The fragment having proB74 and proA was ligated with a DNA fragment as obtained by digesting a plasmid pSTV29 (produced by Takara Shuzo Co.) with Hind III and Bam HI, using a DNA ligation kit (produced by Takara Shuzo Co.) to construct a plasmid having proB74 and proA.

E. coli JM109 strain was transformed with the plasmid obtained above in a usual manner, and the resulting transformant cells were spread on an LB-agar medium comprising 30 µg/ml chloramphenicol and 0.1 mM IPTG, 40 µg/ml X-Gal, and cultivated at 37°C overnight.

A plasmid was extracted in a usual manner from the strain that had produced white colonies through the cultivation, and the structure of the plasmid was identified by digestion with restriction enzyme.

After the process mentioned above, obtained was a plasmid pPF1 having a desensitized gene involved in a fused protein composed of γ-glutamyl kinase with N-terminal, eight amino acid residues (lacZ.Nterm) of the α-fragment of β-galactosidase as fused to its N-terminal, and having a gene proA under the control of Plac (see Fig. 5).

The nucleotide sequence and the amino acid sequence of the structural gene (lacZ.Nterm-proB74) of the fused protein are indicated by Sequence No. 8.

### Example 4 : Production of Trans-4-hydroxy-L-proline by Transformant Having Proline Biosynthesis-gene Expressing Plasmid:

The strain E. coli WT1 as prepared in Example 2 was transformed with the plasmid pPF1 as constructed in Example 3, to obtain a transformant E. coli WT1/pPF1.

The strain WT1 as prepared in Example 2 was transformed with the proline 4-hydroxylase-expressing plasmid pWFH1 as constructed in Example 1, to obtain a transformant E. coli WT1/pWFH1.

Competent cells of the transformant E. coli WT1/pWFH1 were prepared according to the calcium chloride method, into which was introduced the plasmid pPF1 that had been produced in Example 3. Thus a transformant E. coli WT1/pWFH1/pPF1 having the two plasmids was obtained through the selection of the colonies as growing on an LB medium containing 30 µg/ml of chloramphenicol and 50 µg/ml of ampicillin.

Strains of WT1, WT1/pPF1, WT1/pWFH1 and WT1/pWFH1/pPF1 were separately cultivated in an LB medium each comprising 37.5 µg/ml kanamycin, 100 µg/ml ampicillin, 30 µg/ml chloramphenicol, or both 100 µg/ml ampicillin and 30 µg/ml chloramphenicol at 37°C for 16 hours.

Then 100 µl of each culture was inoculated in a test tube (ø 20 x 200 mm) filled with 10 ml of a Med7 medium (comprising 2 % glucose, 1 % ammonium sulfate, 0.1% K₂HPO₄, 0.2% NaCl, 0.05 % MgSO₄, 0.0278 % FeSO₄, 0.0015 % CaCl₂ and 0.8 % peptone)containing 2 % (w/v) calcium carbonate, and cultivated at 30°C for 24 hours.

The amount of L-proline and that of trans-4-hydroxy-L-proline in the culture supernatant are shown in Table 2.

**Table 2**

| Strain (g/l) | L-proline (g/l) | Trans-4-hydroxy-L-proline |
|---|---|---|
| E . coli WT1 | 0.05 | 0.00 |
| E . coli WT1/pPF1 | 1.20 | 0.00 |
| E . coli WT1/pWFH1 | 0.00 | 0.07 |
| E . coli WT1/pWFH1/pPF1 | 0.20 | 0.67 |

It is known from the above data that the transformants having the proline biosynthesis-gene expressing plasmid pPF1 produced a larger amount of L-proline than the others and that the transformant having both the L-proline 4-hydroxylase-expressing plasmid pWFH1 and the proline biosynthesis-gene expressing plasmid pPF1 produced a larger amount of trans-4-hydroxy-L-proline than the transformant having only the L-proline 4-hydroxylase-expressing plasmid pWFH1.

### Example 5 : Construction of Plasmid Expressing L-proline-4-hydroxylase Gene and Proline Biosynthesis-Genes proB74 and proA:

A plasmid pWFP1 was constructed according to the method mentioned below, the plasmid carrying capable of expressing all of a Dactylosporangium sp. RH1-derived L-proline-4-hydroxylase gene and genes proB74 and proA.

The structural gene of L-proline-4-hydroxylase was amplified through PCR, using pWFH1 that had been produced in Example 1, as a template.

For the reaction, used was 20 µl of a reaction mixture comprising 0.1 µg of pWFH1, 0.5 U Pfu DNA polymerase (produced by STRATAGENE Co.), 2 µl of x10 buffer for Pfu DNA polymerase (produced by STRATAGENE Co.), 2 µl of DMSO, 1 µl of 2.5 mM dNTP, 2 µM of the synthetic DNA as indicated in Sequence No. 9 and 2 µM of the synthetic DNA as indicated in Sequence No. 10. Prior to the subsequent cycle reaction, the reaction mixture was pre-incubated at 96°C for 5 minutes. A three step incubation, namely at 96°C for 2 minutes, at 58°C for 1 minutes and at 75°C for 3 minute was repeated for a total of 30 times.

After thus reacted, the reaction mixture was subjected to agarose gel electrophoresis, through which was extracted an amplified fragment of about 800 pb having an L-proline-4-hydroxylase gene in a usual manner. The DNA fragment was collected, using GENECLEAN II KIT (produced by BIO 101, Inc.).

The thus-collected DNA fragment was cleaved with Hind III and Eco RI and then subjected to agarose gel electrophoresis, through which was collected a DNA fragment using GENECLEAN II KIT (produced by BIO 101, Inc.).

The thus-collected, L-proline-4-hydroxylase gene fragment was ligated with a DNA fragment as obtained through digestion of a plasmid pBluescriptII KS(+) (produced by STRATAGENE Co.) with Hind III and Eco RI, using a DNA ligation kit (produced by Takara Shuzo Co.), to thereby construct a plasmid pBII-4OH having an L-proline-4-hydroxylase fragment as inserted thereinto (see Fig. 6).

Genes proB74 and proA were amplified through PCR, using the pPRO74 that had been produced in Example 3, as a template.

For the reaction, used was 20 µl of a reaction mixture comprising 0.1 µg of pPRO74, 1 U of Takara Ex Taq (produced by Takara Shuzo Co. - Code RR001Q), 2 µl of x10 buffer for Takara Ex Taq (produced by Takara Shuzo Co.), 1.6 µl of 2.5 mM dNTP, 2 µM of the synthetic DNA of Sequence No. 11 and 2 µM of the synthetic DNA of Sequence No. 12. A three step incubation, namely at 94°C for 1 minute, at 42°C for 2 minutes and at 75°C for 3 minute was repeated for a total of 30 times.

After thus reacted, the reaction mixture was subjected to agarose gel electrophoresis, through which was extracted an amplified fragment of about 2.3 kbp having genes proB74 and proA in a usual manner. The DNA fragment was collected, using GENECLEAN II KIT (produced by BIO 101, Inc.). The thus-collected DNA fragment was cleaved with Bam HI and Eco RI, then treated with phenol/chloroform, and precipitated with ethanol, and the DNA fragment was collected. The thus-collected DNA fragment having genes proB74 and proA was ligated with a DNA fragment as obtained through digestion of the plasmid pBII-4OH with Hind III and Eco RI, using a DNA ligation kit (produced by Takara Shuzo Co.), to thereby construct a plasmid pBII-4OHBA having L-proline-4-hydroxylase gene and genes proB74 and proA (see Fig. 7).

The plasmid pBII-4OHBA was cleaved with Hind III and Bam HI, and then subjected to agarose gel electrophoresis, through which was collected a DNA fragment of about 3.16 kbp having L-proline-4-hydroxylase gene and genes proB74 and proA. On the other hand, pWFH1 that had been produced in Example 1 was cleaved with Hind III and Bam HI, and then subjected to agarose gel electrophoresis, through which was collected a DNA fragment of about 2.6 kbp not having L-proline-4-hydroxylase gene but having a replication-starting point. These two DNA fragments thus obtained were ligated, using a DNA ligation kit (produced by Takara Shuzo Co.), to thereby construct a plasmid pWFP1 capable of expressing L-proline-4-hydroxylase, proB74 protein and proA protein under the control of a tryptophan tandem promoter (see Fig. 8).

### Example 6 : Production of Trans-4-hydroxy-L-proline by Transformants E. coli WT1/pWFH1/pPF1 and E. coli WT1/pWFP1

The transformant WT1/pWFH1/pPF1 was inoculated in 50 ml of a Med4G medium [comprising 2 % of glucose, 1 % of polypeptone (produced by Nippon Seiyaku KK), 0.5 % of yeast extract (produced by Difco Co.), 1 % of NaCl, 2 % of calcium carbonate, pH 7.0] containing 100 µg/ml ampicillin and 30 µg/ml chloramphenicol, the transformant WT1/pWFP1 was inoculated in 50 ml of a Med4G medium containing 100 µg/ml ampicillin, and cultivated at 30°C for 16 hours with shaking.

The resulting cultures were separately used as seed cultures and inoculated in 5-liter jar fermenters filled with 2 liters of Med7 medium. For the transformant WT1/pWFH1/pPF1, 100 µg/ml ampicillin and 30 µg/ml chloramphenicol were added, and for the transformant WT1/pWFP1, 100 µg/ml ampicillin was added. The transformants in the culture were cultivated under the condition of 400 rpm and 1 vvm, at 30°C.

For the transformant WT1/pWFH1/pPF1, at 8 hours after the start of cultivation, IPTG was added to the medium so as to make the IPTG concentration of 0.2 mM.

For both the transformants, at 24 hours after the start of cultivation, ampicillin and chloramphenicol were added to the medium so as to make ampicillin concentration of 100 µg/ml and the chloramphenicol concentration of 30 µg/ml.

During the cultivation, glucose was suitably added to the medium so as to make the glucose concentration of about 1 %, and the lowermost limit of the pH of the medium was controlled at 6.5 by adding NH₄OH to the medium. Five hours after the start of the cultivation, the concentration of the dissolved oxygen in the culture was controlled to be 1/15 of that at the start of the cultivation by varying the stirring speed within the range between 250 rpm and 700 rpm.

Ninety nine hours after the start of the cultivation, the culture were centrifuged, and the amount of trans-4-hydroxy-L-proline in the supernatants were quantitatively determined. In the supernatant of the culture of E. coli WT1/pWFH1/pPF1, 156 mM (20.5 g/l) of trans-4-hydroxy-L-proline was produced and accumulated. In a supernatant of the culture of E. coli WT1/pWFP1, 191 mM (25.0 g/l) of trans-4-hydroxy-L-proline was produced and accumulated.

### Example 7 : Production of Trans-4-hydroxy-L-proline by Transformant Having Plasmid Expressing L-proline 4-Hydroxylase Gene and Proline Biosynthesis-Gene

The strain E. coli WT1 as prepared in Example 2 was transformed with the plasmid pWFP1 as constructed in Example 5, to obtain a transformant E. coli WT1/pWFP1.

The transformant E. coli WT1/pWFP1 was cultivated in an LB medium containing 100 µg/ml ampicillin, at 37°C for 16 hours. Then 100 µl of the culture was inoculated in a test tube (Ø 20 200 mm) filled with 10 ml of a Med7 medium containing 2 % (w/v) of calcium carbonate, and cultivated therein at 30°C for 24 hours.

The amount of L-proline in a supernatant of the culture was 0.56 g/liter, and that of trans-4-hydroxy-L-proline in the same was 2.4 g/liter.

### Example 8 : Conversion of L-proline into Trans-4-hydroxy-L-proline with Transformant cells:

Cells of the transformant E. coli ATCC12435/pTr2-4OH were inoculated in 3 ml of an LB medium containing 50 µg/ml ampicillin and cultivated therein overnight at 30°C with shaking. The culture was centrifuged to collect wet cells of E. coli ATCC12435/pTr2-4OH.

The transformant E. coli ATCC12435/pWFH1 was inoculated in 100 ml of a Med4 medium [comprising 1 % of polypeptone (produced by Nippon Seiyaku KK), 0.5 % of yeast extract (produced by Difco Co.), 0.5 % of NaCl] containing 100 µg/ml ampicillin, and cultivated therein at 30°C for 16 hours with shaking.

The resulting cultures were separately inoculated in 5-liter jar fermenters filled with 2 liters of Med7 medium (comprising 2 % glucose, 1 % ammonium sulfate, 0.1 % K₂HPO₄, 0.2 % NaCl, 0.05 % MgSO₄, 0.0278 % FeSO₄, 0.0015 % CaCl₂ and 0.8 % peptone), to which were added 200 mM of L-Pro, and cultivated under the condition of 1 vvm, at 33°C for 48 hours.

When the concentration of the dissolved oxygen in the culture became 1/15 comparison to that of the start of the cultivation, the concentration of the dissolved oxygen in the culture was controlled to be 1/15 of that at the start of the cultivation by varying the stirring speed under the base condition of 400 rpm/min and upper condition of 700 rpm/min.

During the cultivation, glucose was suitably added to the medium so as not to lack the glucose, L-Proline was suitably added to the medium so as to make the L-Proline concentration of about 50 mM, and the lowermost limit of the pH of the medium was controlled at 6.5 by adding NH₄OH to the medium.

The cultures were centrifuged to collect wet cells of E. coli ATCC12435/pTr2-4OH and E. coli ATCC12435/pWFH1.

If desired, both of the cells were frozen and stored at -20°C and thawed before use.

The cells were separately added to 250 µl of a reaction mixture (comprising 12 mM L-proline, 24 mM 2-ketoglutaric acid, 4 mM ferrous sulfate and 8 mM L-ascorbic acid in 240 mM MES buffer, pH 6.5) at 4 % (w/v) in terms of the wet cells, and reacted at 35°C for 10 minutes. The reaction was stopped by heating the reaction mixture at 100°C for 2 min.

After the reaction mixtures were centrifuged, the amount of trans-4-hydroxy-L-proline as accumulated in a supernatant was quantitatively determined. In the supernatant of E. coli ATCC12435/pTr2-4OH, 3 mM/l trans-4-hydroxy-L-proline was produced and in the supernatant of E. coli ATCC12435/pWFH1, 16 mM/l trans-4-hydroxy-L-proline was produced. The L-proline-4-hydroxylase activity of these cells were 7.5 and 40 U/mg wet cells respectively.

### Reference Example 1 : Construction of L-proline-4-hydroxylase expression plasmid

A sense strand DNA primer indicated in Sequence No. 13 and an anti-sense strand DNA primer indicated in Sequence No. 14 were synthesized by 38OA^{.}DNA Synthesizer manufactured by Applied Biosystems. The PCR was conducted using the synthetic DNA's as the primers and pRH71 [obtained from Escherichia coli SOLR/pRH71 (FERM BP-5025) containing said plasmid]as a template.

PCR was conducted using 20 µl of a reaction mixture containing 0.1 µg of pRH71, 2 µM sense strand DNA primer, 2 µM anti-sense strand DNA primer, 0.125 U Pfu DNA polymerase (produced by STRATAGENE Co.), 10% DMSO, 20mM Tris-HCl, 10mM KCl, 6mM ammonium sulfate, 2mM MgCl₂, 0.1% TritonX-100 and 10ng/µl Bovine Serum Albumine. After the reaction mixture was incubated at 96°C for 5 minutes, a three step incubation, namely at 96°C for 2 minutes, at 58°C for 1 minute and at 75°C for 1 minute was repeated for a total of 30 times. The reaction mixture was subjected to agarose gel electrophoresis. After it was identified that an amplified fragment of 844 bp encoding the structural gene involved in L-proline-4-hydroxylase was formed, the amplified fragment was extracted from the agarose gel in a usual manner, and recovered using Prep-A-gene produced by Biorad Co. Both terminals of the DNA fragment of 844 bp recovered were cleaved with Hind III and Bam HI, and an ethanol precipitate was then obtained by the ethanol precipitation. The ethanol precipitate was dissolved in 5 µl of TE.

An ATG vector, pTrS32 formed by combining a synthetic linker and plasmid pKYP200, which is composed of a basic plasmid pBR322 together with two promoters Ptrps connected in series (Ptrpx2), was cleaved with Hind III and Bam HI. Hind III-Bam HI fragment containing the structural gene involved in L-proline-4-hydroxylase was inserted into the Hind III-Bam HI cleavage site of the vector, using a ligation kit (produced by Takara Shuzo Co.).

With the thus-obtained plasmid, E. coli XL1-Blue MRF' strain were transformed in a usual manner. The transformant was spread on LB-agar medium containing 50 µg/ml ampicillin and then cultivated thereon overnight at 37°C. The plasmid was extracted from grown colonies of the transformant cells in a usual manner, and the structure of the plasmid was identified by digestion with restriction enzyme. The part of the structural gene of L-proline-4-hydroxylase was sequenced to determine its nucleotide sequence, using a base sequencing kit (Taq DyeDeoxy™ Terminator Cycle Sequencing Kit, produced by Applied Biosystems Co.). The determined nucleotide sequence of the structural gene is indicated by Sequence No. 15.

As a result, plasmid pTr2-4OH in which the DNA fragment encoding the structural gene involved in L-proline-4-hydroxylase was inserted in the same direction as the transcription direction of Ptrpx2 was obtained shown in Fig. 9.

### Reference Example 2 : Production of Trans-4-hydroxy-L-proline by Dactylosporangium sp. RH1:

SR3 medium comprising 1.0% glucose, 1.0% soluble starch, 0.5% yeast extract, 0.5% tryptone, 0.3% meat extract and 0.05% magnesium phosphate was adjusted to pH 7.2 with 6N NaOH, was put in test tubes in an amount of 10 ml each and sterilized at 120°C for 20 minutes. One loopful of cells of Dactylosporangium sp. RH1, that had grown in HT-agar plate medium, was inoculated into the above-mentioned SR3 medium in each test tube, cultivated at 28°C for 2 days with shaking. The resulting culture was used as a seed culture in the following steps.

Separately, Df1 medium comprising 5% soluble starch, 1.5% soybean meal, 0.05% monopotassium phosphate, 0.05% magnesium sulfate 7 hydrate and 0.5% calcium carbonate, and adjusted to pH 7.0 with 6N NaOH, was put in test tubes (diameter 25 mm x length 200 mm) in an amount of 10 ml each and sterilized at 120°C for 20 minutes. One ml of the above-mentioned seed culture was inoculated in the medium in each test tube under germ-free condition and cultivated at 28°C for 2 days with shaking.

The thus-obtained culture was centrifuged at 8000 x g for 10 minutes at 4°C. The cells thus separated were washed with 80 mM TES [N-tris(hydroxymethyl)methyl-2-aminoethanesulfonic acid] buffer (pH 7.5) and then recentrifuged.

In 1.5 ml of a reaction mixture which had been prepared by adding 1.4% (v/v) of Nymeen solution [prepared by adding 4g of Nymeen S-215 (produced by Nippon Oils & Fats Co.) to 10 ml of xylene] to 80 mM TES buffer (pH 7.5) containing 4 mM L-proline, 8 mM 2-ketoglutaric acid, 4 mM L-ascorbic acid and 2 mM ferrous sulfate, 150 mg of the thus-obtained wet cells was suspended, and the mixture was allowed to stand at 30°C for 30 minutes to carry out the enzymatic reaction.

After the reaction, the cells were removed from the reaction mixture by centrifugation, and the amount of trans-4-hydroxy-L-proline accumulated in the supernatant was determined.

As a result of the determination, it was verified that 84 µmol/l trans-4-hydroxy-L-proline was produced in the reaction mixture. The L-proline-4-hydroxylase activity of said cells was 0.028 U/mg wet cells.

### SEQUENCE LISTING

### GENERAL INFORMATION

APPLICANT:
   NAME: KYOWA HAKKO KOGYO CO., LTD.
   STREET: 6-1, Ohtemachi I-chome, Chiyoda-ku
   CITY: Tokyo
   COUNTRY: Japan
   ZIP: 100
TITLE OF INVENTION: Process for producing trans-4-hydroxy-L-proline
NUMBER OF SEQUENCES: 15
COMPUTER READABLE FORM:
   MEDIUM TYPE: 3.5'' Diskette, 1.4 Mb
   COMPUTER: IBM PC compatible
   OPERATING SYSTEM: Windows 95
   SOFTWARE: Word for Windows 6.0
CURRENT APPLICATION DATA:
   APPLICATION NUMBER:
   FILING DATE:
PRIOR APPLICATION DATA:
   APPLICATION NUMBER: JP232724/96
   FILING DATE: September 3, 1996
INFORMATION FOR SEQ ID NO:1:
   SEQUENCE CHARACTERISTICS:
   LENGTH: 816 base pairs
   TYPE: nucleic acid
STRANDEDNESS: double
   MOLECULE TYPE: Other nucleic acid
FEATURE:
   IDENTIFICATION METHOD: by Example
SEQUENCE DESCRIPTION: SEQ ID NO: 1: 
ATG CTG ACC CCG ACG GAG CTC AAG CAG TAC CGC GAG GCG GGC TAT CTG 48
Met Leu Thr Pro Thr Glu Leu Lys Gln Tyr Arg Glu Ala Gly Tyr Leu
1               5                   10                  15
CTC ATC GAG GAC GGC CTC GGC CCG CGG GAG GTC GAC TGC CTG CGC CGG 96
Leu Ile Glu Asp Gly Leu Gly Pro Arg Glu Val Asp Cys Leu Arg Arg
            20                  25                  30
GCG GCG GCG GCC CTC TAC GCG CAG GAC TCG CCG GAC CGC ACG CTG GAG 144
Ala Ala Ala Ala Leu Tyr Ala Gln Asp Ser Pro Asp Arg Thr Leu Glu
        35                  40                  45
AAG GAC GGC CGC ACC GTG CGC GCG GTC CAC GGC TGC CAC CGG CGC GAC 192
Lys Asp Gly Arg Thr Val Arg Ala Val His Gly Cys His Arg Arg Asp
    50                  55                  60
 
CCG GTC TGC CGC GAC CTG GTC CGC CAC CCG CGC CTG CTG GGC CCG GCG 240
Pro Val Cys Arg Asp Leu Val Arg His Pro Arg Leu Leu Gly Pro Ala
65                  70                  75                  80
ATG CAG ATC CTG TCC GGC GAC GTG TAC GTC CAC CAG TTC AAG ATC AAC 288
Met Gln Ile Leu Ser Gly Asp Val Tyr Val His Gln Phe Lys Ile Asn
                85                  90                  95
GCG AAG GCC CCG ATG ACC GGC GAT GTC TGG CCG TGG CAC CAG GAC TAC 336
Ala Lys Ala Pro Met Thr Gly Asp Val Trp Pro Trp His Gln Asp Tyr
            100                 105                 110
ATC TTC TGG GCC CGA GAG GAC GGC ATG GAC CGT CCG CAC GTG GTC AAC 384
Ile Phe Trp Ala Arg Glu Asp Gly Met Asp Arg Pro His Val Val Asn
        115                 120                 125
GTC GCG GTC CTG CTC GAC GAG GCC ACC CAC CTC AAC GGG CCG CTG TTG 432
Val Ala Val Leu Leu Asp Glu Ala Thr His Leu Asn Gly Pro Leu Leu
    130                 135                 140
TTC GTG CCG GGC ACC CAC GAG CTG GGC CTC ATC GAC GTG GAG CGC CGC 480
Phe Val Pro Gly Thr His Glu Leu Gly Leu Ile Asp Val Glu Arg Arg
145                 150                 155                 160
GCG CCG GCC GGC GAC GGC GAC GCG CAG TGG CTG CCG CAG CTC AGC GCC 528
Ala Pro Ala Gly Asp Gly Asp Ala Gln Trp Leu Pro Gln Leu Ser Ala
                165                 170                 175
GAC CTC GAC TAC GCC ATC GAC GCC GAC CTG CTG GCC CGG CTG ACG GCC 576
Asp Leu Asp Tyr Ala Ile Asp Ala Asp Leu Leu Ala Arg Leu Thr Ala
            180                 185                 190
GGG CGG GGC ATC GAG TCG GCC ACC GGC CCG GCG GGC TCG ATC CTG CTG 624
Gly Arg Gly Ile Glu Ser Ala Thr Gly Pro Ala Gly Ser Ile Leu Leu
        195                 200                 205
TTC GAC TCC CGG ATC GTG CAC GGC TCG GGC ACG AAC ATG TCG CCG CAC 672
Phe Asp Ser Arg Ile Val His Gly Ser Gly Thr Asn Met Ser Pro His
    210                 215                 220
CCG CGC GGC GTC GTC CTG GTC ACC TAC AAC CGC ACC GAC AAC GCC CTG 720
Pro Arg Gly Val Val Leu Val Thr Tyr Asn Arg Thr Asp Asn Ala Leu
225                 230                 235                 240
CCG GCG CAG GCC GCT CCG CGC CCG GAG TTC CTG GCC GCC CGC GAC GCC 768
Pro Ala Gln Ala Ala Pro Arg Pro Glu Phe Leu Ala Ala Arg Asp Ala
                245                 250                 255
ACC CCG CTG GTG CCG CTG CCC GCG GGC TTC GCG CTG GCC CAG CCC GTC 816
Thr Pro Leu Val Pro Leu Pro Ala Gly Phe Ala Leu Ala Gln Pro Val
            260                 265                 270
INFORMATION FOR SEQ ID NO:2:
SEQUENCE CHARACTERISTICS:
   LENGTH: 64 base pairs
   TYPE: nucleic acid
TOPOLOGY: linear
   MOLECULE TYPE: other nucleic acid, synthetic DNA
SEQUENCE DESCRIPTION: SEQ ID NO: 2: 
TGAGGAAAGC TTATGCTGAC CCCGACCGAA CTGAAACAGT ATCGTGAAGC 50
GGGCTATCTG CTGA 64
INFORMATION FOR SEQ ID NO:3:
SEQUENCE CHARACTERISTICS:
   LENGTH: 66 base pairs
   TYPE: nucleic acid
TOPOLOGY: linear
   MOLECULE TYPE: other nucleic acid, synthetic DNA
SEQUENCE DESCRIPTION: SEQ ID NO:3: 
CCGGAATTCG TCGACTTCAC GCGGGCCCAG GCCATCTTCA ATCAGCAGAT 50
AGCCCGCTTC ACGATA 66
INFORMATION FOR SEQ ID NO:4:
SEQUENCE CHARACTERISTICS:
   LENGTH: 22 base pairs
   TYPE: nucleic acid
TOPOLOGY: linear
   MOLECULE TYPE: other nucleic acid, synthetic DNA
SEQUENCE DESCRIPTION: SEQ ID NO:4: GACCCGTGCT AATATGGAAG AC 22
INFORMATION FOR SEQ ID NO:5:
SEQUENCE CHARACTERISTICS
   LENGTH: 22 base pairs
   TYPE: nucleic acid
TOPOLOGY: linear
   MOLECULE TYPE: other nucleic acid, synthetic DNA
SEQUENCE DESCRIPTION: SEQ ID NO:5: GTCTTCCATA TTAGCACGGG TC 22
INFORMATION FOR SEQ ID NO:6:
SEQUENCE CHARACTERISTICS
   LENGTH: 33 base pairs
   TYPE: nucleic acid
TOPOLOGY: linear
   MOLECULE TYPE: other nucleic acid, synthetic DNA
SEQUENCE DESCRIPTION: SEQ ID NO:6: GGCAAAGCTT CATGAGTGAC AGCCAGACGC TGG 33
INFORMATION FOR SEQ ID NO:7:
SEQUENCE CHARACTERISTICS
   LENGTH: 33 base pairs
   TYPE: nucleic acid
TOPOLOGY: linear
   MOLECULE TYPE: other nucleic acid, synthetic DNA
SEQUENCE DESCRIPTION: SEQ ID NO:7: TTTGCAGGAT CCGGTTTTAT TTACGCACGA ATG 33
INFORMATION FOR SEQ ID NO:8:
SEQUENCE CHARACTERISTICS
   LENGTH: 1125 base pairs
   TYPE: nucleic acid
TOPOLOGY: double
   MOLECULE TYPE: other nucleic acid
SEQUENCE DESCRIPTION: SEQ ID NO:8: 
ATG ACC ATG ATT ACG CCA AGC TTC ATG AGT GAC AGC CAG ACG CTG GTG 48
Met Thr Met Ile Thr Pro Ser Phe Met Ser Asp Ser Gln Thr Leu Val
1               5                   10                  15
GTA AAA CTC GGC ACC AGT GTG CTA ACA GGC GGA TCG CGC CGT CTG AAC 96
Val Lys Leu Gly Thr Ser Val Leu Thr Gly Gly Ser Arg Arg Leu Asn
            20                  25                  30
CGT GCC CAT ATC GTT GAA CTT GTT CGC CAG TGC GCG CAG TTA CAT GCC 144
Arg Ala His Ile Val Glu Leu Val Arg Gln Cys Ala Gln Leu His Ala
        35                  40                  45
GCC GGG CAT CGG ATT GTT ATT GTG ACG TCG GGC GCG ATC GCC GCC GGA 192
Ala Gly His Arg Ile Val Ile Val Thr Ser Gly Ala Ile Ala Ala Gly
    50                  55                  60
CGT GAG CAC CTG GGT TAC CCG GAA CTG CCA GCG ACC ATC GCC TCG AAA 240
Arg Glu His Leu Gly Tyr Pro Glu Leu Pro Ala Thr Ile Ala Ser Lys
65                  70                  75                  80
CAA CTG CTG GCG GCG GTA GGG CAG AGT CGA CTG ATT CAA CTG TGG GAA 288
Gln Leu Leu Ala Ala Val Gly Gln Ser Arg Leu Ile Gln Leu Trp Glu
                85                  90                  95
CAG CTG TTT TCG ATT TAT GGC ATT CAC GTC GGG CAA ATG CTG CTG ACC 336
Gln Leu Phe Ser Ile Tyr Gly Ile His Val Gly Gln Met Leu Leu Thr
            100                 105                 110
CGT GCT AAT ATG GAA GAC CGT GAA CGC TTC CTG AAC GCC CGC GAC ACC 384
Arg Ala Asn Met Glu Asp Arg Glu Arg Phe Leu Asn Ala Arg Asp Thr
        115                 120                 125
CTG CGA GCG TTG CTC GAT AAC AAT ATC GTT CCG GTA ATC AAT GAG AAC 432
Leu Arg Ala Leu Leu Asp Asn Asn Ile Val Pro Val Ile Asn Glu Asn
 
130                 135                 140
GAT GCT GTC GCT ACG GCA GCG ATT AAG GTC GGC GAT AAC GAT AAC CTT      480
Asp Ala Val Ala Thr Ala Ala Ile Lys Val Gly Asp Asn Asp Asn Leu
145                 150                 155                 160
TCT GCG CTG GCG GCG ATT CTT GCG GGT GCC GAT AAA CTG TTG CTG CTG      528
Ser Ala Leu Ala Ala Ile Leu Ala Gly Ala Asp Lys Leu Leu Leu Leu
                165                 170                 175
ACC GAT CAA AAA GGT TTG TAT ACC GCT GAC CCG CGC AGC AAT CCG CAG      576
Thr Asp Gln Lys Gly Leu Tyr Thr Ala Asp Pro Arg Ser Asn Pro Gln
            180                 185                 190
GCA GAA CTG ATT AAA GAT GTT TAC GGC ATT GAT GAC GCA CTG CGC GCG      624
Ala Glu Leu Ile Lys Asp Val Tyr Gly Ile Asp Asp Ala Leu Arg Ala
        195                 200                 205
ATT GCC GGT GAC AGC GTT TCA GGC CTC GGA ACT GGC GGC ATG AGT ACC      672
Ile Ala Gly Asp Ser Val Ser Gly Leu Gly Thr Gly Gly Met Ser Thr
    210                 215                 220
AAA TTG CAG GCC GCT GAC GTG GCT TGC CGT GCG GGT ATC GAC ACC ATT      720
Lys Leu Gln Ala Ala Asp Val Ala Cys Arg Ala Gly Ile Asp Thr Ile
225                 230                 235                 240
ATT GCC GCG GGC AGC AAG CCG GGC GTT ATT GGT GAT GTG ATG GAA GGC      768
Ile Ala Ala Gly Ser Lys Pro Gly Val Ile Gly Asp Val Met Glu Gly
                245                 250                 255
ATT TCC GTC GGT ACG CTG TTC CAT GCC CAG GCG ACT CCG CTT GAA AAC      816
Ile Ser Val Gly Thr Leu Phe His Ala Gln Ala Thr Pro Leu Glu Asn
            260                 265                 270
CGT AAA CGC TGG ATT TTC GGT GCG CCG CCG GCG GGT GAA ATC ACG GTA      864
Arg Lys Arg Trp Ile Phe Gly Ala Pro Pro Ala Gly Glu Ile Thr Val
        275                 280                 285
GAT GAA GGG GCA ACT GCC GCC ATT CTG GAA CGC GGC AGC TCC CTG TTG      912
Asp Glu Gly Ala Thr Ala Ala Ile Leu Glu Arg Gly Ser Ser Leu Leu
    290                 295                 300
CCG AAA GGC ATT AAA AGC GTG ACT GGC AAT TTC TCG CGT GGT GAA GTC      960
Pro Lys Gly Ile Lys Ser Val Thr Gly Asn Phe Ser Arg Gly Glu Val
305                 310                 315                 320
ATC CGC ATT TGC AAC CTC GAA GGC CGC GAT ATC GCC CAC GGC GTC AGT     1008
Ile Arg Ile Cys Asn Leu Glu Gly Arg Asp Ile Ala His Gly Val Ser
                325                 330                 335
CGT TAC AAC AGC GAT GCA TTA CGC CGT ATT GCC GGA CAC CAC TCG CAA     1056
Arg Tyr Asn Ser Asp Ala Leu Arg Arg Ile Ala Gly His His Ser Gln
            340                 345                 350
GAA ATT GAT GCA ATA CTG GGA TAT GAA TAC GGC CCG GTT GCC GTT CAC     1104
Glu Ile Asp Ala Ile Leu Gly Tyr Glu Tyr Gly Pro Val Ala Val His
        355                 360                 365
CGT GAT GAC ATG ATT ACC CGT                                         1125
Arg Asp Asp Met Ile Thr Arg
    370                 375
INFORMATION FOR SEQ ID NO:9:
SEQUENCE CHARACTERISTICS:
   LENGTH: 37 base pairs
   TYPE: nucleic acid
TOPOLOGY: linear
   MOLECULE TYPE: other nucleic acid, synthetic DNA
SEQUENCE DESCRIPTION: SEQ ID NO: 9: TATCGATAAG CTTATGCTGA CCCCGACCGA ACTGAAA 37
INFORMATION FOR SEQ ID NO:10:
SEQUENCE CHARACTERISTICS:
   LENGTH: 33 base pairs
   TYPE: nucleic acid
TOPOLOGY: linear
   MOLECULE TYPE: other nucleic acid, synthetic DNA
SEQUENCE DESCRIPTION: SEQ ID NO:10: GGCAGAATTC TAGACGGGCT GGGCCAGCGC GAA 33
INFORMATION FOR SEQ ID NO:11:
SEQUENCE CHARACTERISTICS:
   LENGTH: 38 base pairs
   TYPE: nucleic acid
TOPOLOGY: linear
   MOLECULE TYPE: other nucleic acid, synthetic DNA
SEQUENCE DESCRIPTION: SEQ ID No:11: AAAATTGAAT TCCAGAGAAT CATGAGTGAC AGCCAGAC 38
INFORMATION FOR SEQ ID NO:12:
SEQUENCE CHARACTERISTICS:
   LENGTH: 36 base pairs
   TYPE: nucleic acid
TOPOLOGY: linear
   MOLECULE TYPE: other nucleic acid, synthetic DNA
SEQUENCE DESCRIPTION: SEQ ID No:12: ACCCGGATCC ATTTACGCAC GAATGGTGTA ATCACC 36
INFORMATION FOR SEQ ID NO:13:
SEQUENCE CHARACTERISTICS:
   LENGTH: 37 base pairs
   TYPE: nucleic acid
TOPOLOGY: linear
   MOLECULE TYPE: other nucleic acid, synthetic DNA
SEQUENCE DESCRIPTION: SEQ ID No:13: GTGAGGAAAG CTTATGCTGA CCCCGACGGA GCTCAAG 37
INFORMATION FOR SEQ ID No:14:
SEQUENCE CHARACTERISTICS:
   LENGTH: 36 base pairs
   TYPE: nucleic acid
TOPOLOGY: linear
   MOLECULE TYPE: other nucleic acid, synthetic DNA
SEQUENCE DESCRIPTION: SEQ ID No:14: GCCTGCGGGA TCCTAGACGG GCTGGGCCAG CGCGAA 36
INFORMATION FOR SEQ ID NO:15:
SEQUENCE CHARACZERISTICS:
   LENGTH: 816 base paires
   TYPE: nucleic acid
STRANDEDNESS: double
   MOLECULE TYPE: genomic DNA
ORIGINAL SOURCE:
   ORGANISM: Dactylosporangium sp.
   STRAIN: RH1
FEATURE:
   IDENTIFICATION METHOD: by Example
SEQUENCE DESCRIPTION: SEQ ID No:15:

ATGCTGACCC CGACGGAGCT CAAGCAGTAC CGCGAGGCGG GCTATCTGCT CATCGAGGAC 60
GGCCTCGGCC CGCGGGAGGT CGACTGCCTG CGCCGGGCGG CGGCGGCCCT CTACGCGCAG 120
GACTCGCCGG ACCGCACGCT GGAGAAGGAC GGCCGCACCG TGCGCGCGGT CCACGGCTGC 180
CACCGGCGCG ACCCGGTCTG CCGCGACCTG GTCCGCCACC CGCGCCTGCT GGGCCCGGCG 240
ATGCAGATCC TGTCCGGCGA CGTGTACGTC CACCAGTTCA AGATCAACGC GAAGGCCCCG 300
ATGACCGGCG ATGTCTGGCC GTGGCACCAG GACTACATCT TCTGGGCCCG AGAGGACGGC 360
ATGGACCGTC CGCACGTGGT CAACGTCGCG GTCCTGCTCG ACGAGGCCAC CCACCTCAAC 420
GGGCCGCTGT TGTTCGTGCC GGGCACCCAC GAGCTGGGCC TCATCGACGT GGAGCGCCGC 480
GCGCCGGCCG GCGACGGCGA CGCGCAGTGG CTGCCGCAGC TCAGCGCCGA CCTCGACTAC 540
GCCATCGACG CCGACCTGCT GGCCCGGCTG ACGGCCGGGC GGGGCATCGA GTCGGCCACC 600
GGCCCGGCGG GCTCGATCCT GCTGTTCGAC TCCCGGATCG TGCACGGCTC GGGCACGAAC 660
ATGTCGCCGC ACCCGCGCGG CGTCGTCCTG GTCACCTACA ACCGCACCGA CAACGCCCTG 720
CCGGCGCAGG CCGCTCCGCG CCCGGAGTTC CTGGCCGCCC GCGACGCCAC CCCGCTGGTG 780
CCGCTGCCCG CGGGCTTCGC GCTGGCCCAG CCCGTC 816

## Claims

1. A non-human transformant having a vector comprising a DNA fragment that contains a gene coding for a protein which has the enzymatic activity of hydroxylating the 4-position of L-proline and which acts on free L-proline in the presence of 2-ketoglutaric acid and divalent iron ions to produce trans-4-hydroxy-L-proline, and wherein the transformant comprises an increased number of copies of a gene coding for an enzyme participating in the biosynthesis of L-proline, wherein a proline biosynthesis-gene which codes for an enzyme that is subjected to feedback inhibition with proline has been mutated to reduce the feedback inhibition with proline, or wherein the proline decomposition activity has been removed from the host to reinforce the proline biosynthesis activity.

2. The transformant according to claim 1, wherein the gene coding for an enzyme participating in the biosynthesis of the L-proline is *proB* or *proA*.

3. The transformant according to claim 1, wherein the gene which codes for an enzyme that is subjected to feedback inhibition with proline is *proB*74 derived from *E. coli.*

4. A biologically pure culture of *Escherichia coli* WT1 deposited under Ferm BP-5618.

5. A process for producing trans-4-hydroxy-L-proline, which comprises cultivating in a medium a transformant according to any one of claims 1, 2, or 3, allowing L-proline to coexist with 2-ketoglutaric acid and divalent iron ions in an aqueous medium in the presence of cells of the cultivated transformant as an enzyme source, converting L-proline into trans-4-hydroxy-L-proline and recovering the resulting trans-4-hydroxy-L-proline from the aqueous medium.

6. The process for producing trans-4-hydroxy-L-proline according to claim 5, wherein the aqueous medium is a culture medium.

7. The process according to claim 5, wherein a surfactant or an organic solvent is added to the aqueous medium.

8. A non-human transformant according to claim 1 having a vector comprising a DNA fragment that contains a gene coding for a protein which has the enzymatic activity of hydroxylating the 4-position of L-proline and which acts on free L-proline in the presence of 2-ketoglutaric acid and divalent iron ions to produce trans-4-hydroxy-L-proline, wherein the transformant has an ability to produce 1.2g/l L-proline when cultured in LB medium at 37°C for 16 hours.

## Patentansprüche

1. Nicht-menschliche Transformante mit einem Vektor, der ein DNA-Fragment umfasst, das ein Gen enthält, das für ein Protein kodiert, das die enzymatische Aktivität zur Hydroxylierung der 4-Position von L-Prolin besitzt und das auf freies L-Prolin in der Gegenwart von 2-Ketoglutarsäure und divalenten Eisenionen wirkt, um trans-4-hydroxy-L-Prolin zu erzeugen und wobei die Transformante eine erhöhte Anzahl von Kopien eines Gens umfasst, das für ein Enzym kodiert, das an der Biosynthese von L-Prolin beteiligt ist, wobei ein Prolin-Biosynthese-Gen, das für ein Enzym kodiert, das einer Feedback-Inhibition mit Prolin ausgesetzt ist, mutiert wurde, um die Feedback-Inhibition mit Prolin zu verringern oder wobei die Prolin-Abbau-Aktivität von dem Wirt entfernt wurde, um die Prolin-Biosynthese-Aktivität zu verstärken.

2. Transformante nach Anspruch 1, wobei das Gen, das für ein Enzym kodiert, das an der Biosynthese von L-Prolin beteiligt ist, *proB* oder *proA* ist.

3. Transformante nach Anspruch 1, wobei das Gen, das für ein Enzym kodiert, das einer Feedback-Inhibition mit Prolin ausgesetzt ist, von *E. coli* abgeleitetes *proB74* ist.

4. Biologisch reine Kultur von *Escherichia coli* WT1, hinterlegt unter Ferm BP-5618.

5. Verfahren zur Herstellung von trans-4-hydroxy-L-Prolin, umfassend das Kultivieren einer Transformante nach einem der Ansprüche 1, 2 oder 3 in einem Medium, das Ermöglichen, dass L-Prolin mit 2-Ketoglutarsäure und divalenten Eisenionen in einem wässrigen Medium in der Gegenwart der kultivierten Transformante als eine Enzymquelle koexistiert, das Umwandeln von L-Prolin in trans-4-hydroxy-L-Prolin und das Gewinnen des entstehenden trans-4-hydroxy-L-Prolins aus dem wässrigen Medium.

6. Verfahren zur Herstellung von trans-4-hydroxy-L-Prolin nach Anspruch 5, wobei das wässrige Medium ein Kulturmedium ist.

7. Verfahren nach Anspruch 5, wobei ein oberflächenaktives Mittel oder ein organisches Lösungsmittel zu dem wässrigen Medium zugegeben wird.

8. Nicht-menschliche Transformante nach Anspruch 1 mit einem Vektor, der ein DNA-Fragment umfasst, der ein DNA-Fragment umfasst, das ein Gen enthält, das für ein Protein kodiert, das die enzymatische Aktivität zur Hydroxylierung der 4-Position von L-Prolin besitzt und das auf freies L-Prolin in der Gegenwart von 2-Ketoglutarsäure und divalenten Eisenionen wirkt, um trans-4-hydroxy-L-Prolin zu erzeugen, wobei die Transformante eine Fähigkeit besitzt 1,2 g/l L-Prolin zu erzeugen, wenn sie in LB-Medium bei 37 °C für 16 Stunden kultiviert wird.

## Revendications

1. Transformant non humain ayant un vecteur comprenant un fragment d'ADN qui contient un gène codant pour une protéine qui a l'activité enzymatique d'hydroxylation de la position 4 de la L-proline et qui agit sur la L-proline libre en présence d'acide 2-cétoglutarique et d'ions fer divalents pour produire de la trans-4-hydroxy-L-proline, et dans lequel le transformant comprend un nombre accru de copies d'un gène codant pour une enzyme participant à la biosynthèse de L-proline, dans lequel un gène de biosynthèse de la proline qui code pour une enzyme qui subit une rétro-inhibition par la proline a été muté pour réduire la rétro-inhibition par la proline, ou dans lequel l'activité de décomposition de la proline a été éliminée chez l'hôte pour renforcer l'activité de biosynthèse de la proline.

2. Transformant selon la revendication 1, dans lequel le gène codant pour une enzyme participant à la biosynthèse de la L-proline est *proB* ou *proA.*

3. Transformant selon la revendication 1, dans lequel le gène qui code pour une enzyme qui subit une rétro-inhibition par la proline est *proB74* dérivé de *E. coli.*

4. Culture de *Escherischia coli* WT1 biologiquement pure déposée sous Ferm BP-5618.

5. Procédé pour produire de la trans-4-hydroxy-L-proline, qui comprend le fait de cultiver dans un milieu un transformant selon l'une quelconque des revendications 1, 2 ou 3 permettant à la L-proline de coexister avec l'acide 2-cétoglutarique et des ions fer divalents dans un milieu aqueux en présence du transformant cultivé comme source d'enzyme convertissant la L-proline en trans-4-hydroxy-L-proline, et de récupérer la trans-4-hydroxy-L-proline résultante à partir du milieu aqueux.

6. Procédé pour produire de la trans-4-hydroxy-L-proline selon la revendication 5 dans lequel le milieu aqueux est un milieu de culture.

7. Procédé selon la revendication 5, dans lequel un tensio-actif ou un solvant organique est ajouté au milieu aqueux.

8. Transformant non humain selon la revendication 1 ayant un vecteur comprenant un fragment d'ADN qui contient un gène codant pour une protéine qui a l'activité enzymatique d'hydroxylation de la position 4 de la L-proline et qui agit sur la L-proline libre en présence d'acide 2-cétoglutarique et d'ions fer divalents pour produire de la trans-4-hydroxy-L-proline, le transformant ayant la capacité de produire 1,2 g/l de L-proline lorsqu'il est cultivé dans du milieu LB à 37°C pendant 16 heures.
